# EUROPEAN PATENT SPECIFICATION

(11) **EP 3 273 985 B1**
(45) Date of publication and mention of the grant of the patent: **08.06.2022**
(21) Application number: 16769546.9
(22) Date of filing: 22.03.2016
(51) Int. Cl.: A61K 38/00, A61K 38/20, C07K 14/545, C12N 5/071, C12N 5/0775, A61P 17/02, A61K 35/28

(54) **IL-1RA BASED COMPOSITIONS AND TREATMENTS**
IL-1RA BASIERTE ZUSAMMENSETZUNGEN UND BEHANDLUNGEN
COMPOSITIONS ET TRAITEMENTS À BASE D'IL-1RA

(30) Priority: 25.03.2015 US 201562138233 P
(43) Date of publication of application: 31.01.2018
(73) Proprietor: Alfred E. Mann Institute for Biomedical Engineering at the University of Southern California, Los Angeles, CA 90089 (US); University of Southern California, Los Angeles, CA 90015 (US)
(72) Inventor: SHI, Songtao, Conshohoken, PA 19428 (US)
(74) Representative: Müller-Boré & Partner Patentanwälte PartG mbB
(86) International application number: PCT/US2016/023579
(87) International publication number: WO 2016/154201

(56) References cited:
- US-B1- 6 337 072
- L. A. ORTIZ ET AL: "Interleukin 1 receptor antagonist mediates the antiinflammatory and antifibrotic effect of mesenchymal stem cells during lung injury", PROCEEDINGS OF THE NATIONAL ACADEMY OF SCIENCES, vol. 104, no. 26, 26 June 2007 (2007-06-26), pages 11002-11007, XP55317073, US ISSN: 0027-8424, DOI: 10.1073/pnas.0704421104
- CARTER D B ET AL: "PURIFICATION, CLONING, EXPRESSION AND BIOLOGICAL CHARACTERIZATION OD AN INTERLEUKIN-1 RECEPTOR ANTAGONIST PROTEIN", NATURE, MACMILLAN JOURNALS LTD, LONDON, GB, vol. 344, no. 6267, 12 April 1990 (1990-04-12), pages 633-638, XP002038695, ISSN: 0028-0836, DOI: 10.1038/344633A0
- SÉBASTIEN OTTAVIANI ET AL: "Efficacy of anakinra in gouty arthritis: a retrospective study of 40 cases", ARTHRITIS RESEARCH AND THERAPY, BIOMED CENTRAL, LONDON, GB, vol. 15, no. 5, 17 September 2013 (2013-09-17), page R123, XP021173940, ISSN: 1478-6354, DOI: 10.1186/AR4303
- C. QUINIOU ET AL: "Development of a Novel Noncompetitive Antagonist of IL-1 Receptor", THE JOURNAL OF IMMUNOLOGY, vol. 180, no. 10, 15 May 2008 (2008-05-15) , pages 6977-6987, XP055423379, US ISSN: 0022-1767, DOI: 10.4049/jimmunol.180.10.6977
- TOMAR G B ET AL: "Human gingiva-derived mesenchymal stem cells are superior to bone marrow-derived mesenchymal stem cells for cell therapy in regenerative medicine", BIOCHEMICAL AND BIOPHYSICAL RESEARCH COMMUNICATIONS, ELSEVIER, AMSTERDAM, NL, vol. 393, no. 3, 12 March 2010 (2010-03-12), pages 377-383, XP026960849, ISSN: 0006-291X, DOI: 10.1016/J.BBRC.2010.01.126 [retrieved on 2010-02-06]
- "Oral Presentations", JOURNAL OF TISSUE ENGINEERING AND REGENERATIVE MEDICINE, vol. 8, 1 June 2014 (2014-06-01), pages 39-206, XP055165929, ISSN: 1932-6254, DOI: 10.1002/term.1931
- DARWIN J PROCKOP ET AL: "Mesenchymal Stem/Stromal Cells (MSCs): Role as Guardians of Inflammation", MOLECULAR THERAPY, vol. 20, no. 1, 18 October 2011 (2011-10-18), pages 14-20, XP055202750, ISSN: 1525-0016, DOI: 10.1038/mt.2011.211
- THOMAY ET AL.: 'Disruption of Interleukin-1 Signaling Improves the Quality of Wound Healing.' AM J. PATHOL. vol. 174, no. ISSUE, June 2009, pages 2129 - 2136, XP055310431
- ORTIZ ET AL.: 'Interleukin 1 receptor antagonist mediates the antiinflammatory and antifibrotic effect of mesenchymal stem cells during lung injury.' PROC. NATL. ACAD. SCI. vol. 104, no. ISSUE, 26 June 2007, pages 11002 - 11007, XP055317073
- ZHANG ET AL.: 'Human Gingiva-Derived Mesenchymal Stem Cells Elicit Polarization of M2 Macrophages and Enhance Cutaneous Wound Healing.' STEM CELLS. vol. 28, no. ISSUE, 31 October 2010, pages 1856 - 1868, XP055317074
- LAN ET AL.: 'Kinetics and Function of Mesenchymal Stem Cells in Corneal Injury.' INVEST. OPHTHALMOL VIS. SCI. vol. 53, no. ISSUE, 14 June 2012, pages 3638 - 3644, XP055317075

## Description

This disclosure relates to stem cell treatment and IL-1RA treatment. Specifically, the present invention relates to compositions comprising an IL-1RA substitute and at least one human stem cell that expressed IL-1RA, or compositions comprising human IL-1 RA, as well as uses of said compositions in the treatment of a wound and/or in reducing scar formation.

Wound healing is a sophisticated process of tissue repair involving multiple cell types and biological pathways. Mammalian adult cutaneous wound healing is mediated by a fibro-proliferative response with scar formation. Dysregulation of this interactive process (e.g., diabetes) may adversely result in delayed wound healing or hypertrophic scarring (Loots et al., 1998; Gurtner et al., 2008). Especially, if the inflammatory phase is prolonged, the pro-inflammatory mediators will direct the myofibroblasts and fibrocytes in larger scar formation (Rhett et al., 2008). These chronic wounds represent the most significant challenges in current clinical practice with over 50% of them being refractory to the treatment (Summer, 2012).

In contrast, early to mid-gestational fetal cutaneous wound healing is more akin to a scarless regeneration. Previous studies have shown that fetal wounds appear to have mild inflammation with fewer inflammatory cells and lower levels of inflammatory factors (Hu et al., 2014). Intriguingly, there is a common impression among clinicians that oral gingiva/mucosa wound heals faster and with minimal scar formation (Häkkinen et al., 2000) when compared to cutaneous wound healing. More importantly, the healing process shows a similar reduced inflammatory cell infiltration and proinflammatory factor production as the fetal wound healing (Szpaderska et al., 2003; Larjava et al., 2011). These evidences indicate that the outcome of wound healing process is largely affected by inflammatory regulation.

Mesenchymal stem cells (MSCs) are described to be a type of multipotent progenitor cells with ability to self-renewal and to differentiate into multiple cell lineages (Friedenstein et al., 1974; Prockop, 1997). Furthermore, MSCs are shown to have the capability to regulate immune response, which has provided a foundation for the use of MSCs in immune therapies (Uccelli et al., 2008; Le Blanc et al., 2008; Sun et al., 2009; Akiyama et al., 2012). It is generally agreed that local connective tissue fibroblasts are the major source involved in skin wound site repair (R. Higashiyama et al., 2011).

In addition, it is now well documented that the local MSCs such as the stem cells residing in the dermal sheath that surrounds the outside of the hair follicle can also differentiate into fibroblasts after a lesion (C.A. Jahoda et al., 2001 and 2003).

Recently, gingiva/mucosa-derived mesenchymal stem cells (GMSCs) were isolated and characterized. Interestingly, these cells have a distinct neural crest origin, share some traits with fetal cells and show potent immunoregulatory properties suggesting that they may possess specific therapeutic potential (Xu et al., 2013; Fournier et al., 2013).

In this context, Ortiz *et al.* Ortiz, L. A. et al.; PNAS, 104(26); 2007; pp. 11002-11007) describes the anti-inflammatory and anti-fibrotic effect of mesenchymal stem cell interleukin 1 receptor antagonist (IL1-RA) during lung injury. Further, Ottaviani *et al.* (Ottaviani, S. et al.; Arthritis Research & Therapy, 15(R123); 2013; pp. 1-6) describes the efficacy of anakinra in gouty arthritis. Furthermore, Quiniou *et al.* (Quiniou, Ch. et al.; The Journal of Immunology, 180; 2008; pp. 6977-6987) describes the development of a non-competitive antagonist of IL-1 receptor. Moreover, Thomay *et al.* (Thomay, A. A. et al.; The American Journal of Pathology, 174; 2009; pp. 2129-2136) describes an improvement of the quality of wound healing by the disruption of interleukin-1 signaling.

Allan SM, Tyrrell PJ, Rothwell NJ. (2005) Interleukin-1 and neuronal injury. Nat Rev Immunol, 5(8): 629-640.

Loots MA, Lamme EN, Zeegelaar J et al. (1998) Differences in cellular infiltrate and extracellular matrix of chronic diabetic and venous ulcers versus acute wounds. J Invest Dermatol, 111:850-857.

Gurtner GC, Werner S, Barrandon Y, Barrandon Y, Longaker MT. (2008) Wound repair and regeneration. Nature, 453: 314-321.

Rhett JM, Ghatnekar GS, Palatinus JA, O'Quinn M, Yost MJ, Gourdie RG. (2008) Novel therapies for scar reduction and regenerative healing of skin wounds. Trends Biotechnol, 26: 173-180.

Summer EH. (2012) Mesenchymal stem cells: a multimodality option for wound healing. Adv Wound Care, 1: 153-158.

Hu MS, Maan ZN, Wu JC, Rennert RC, Hong WX, Lai TS et al. (2014) Tissue engineering and regenerative repair in wound healing. Ann Biomed Eng, 42(7): 1494-1507.

Häkkinen L, Uitto VJ, Larjava H. (2000) Cell biology of gingival wound healing. Periodontology 2000 24: 127-152.

Szpaderska AM, Zuckerman JD, DiPietro LA. (2003) Differential injury responses in oral mucosal and cutaneous wounds. J Dent Res 82: 621-626.

Glim JE, van Egmond M, Niessen FB, Everts V, Beelen RH (2013) Detrimental dermal wound healing: What can we learn from the oral mucosa? Wound Repair Regen 21: 648-660.

Mak K, Manji A, Gallant-Behm C, Wiebe C, Hart DA, et al. (2009) Scarless healing of oral mucosa is characterized by faster resolution of inflammation and control of myofibroblast action compared to skin wounds in the red Duroc pig model. J Dermatol Sci 56: 168-180.

Larjava H, Wiebe C, Gallant-Behm C, Hart DA, Heino J, Häkkinen L. (2011) Exploring scarless healing of oral soft tissues. J Can Dent Assoc 77: b18.

Dinarello CA. (2010) Anti-inflammatory Agents: Present and Future. Cell. 140: 935-950.

R. Higashiyama, S. Nakao, Y. Shibusawa, O. Ishikawa, T. Moro, K. Mikami, et al. Differential contribution of dermal resident and bone marrow-derived cells to collagen production during wound healing and fibrogenesis in mice. J Invest Dermatol. 131 (2011), pp. 529-536

C.A. Jahoda, J. Whitehouse, A.J. Reynolds, N. Hole. Hair follicle dermal cells differentiate into adipogenic and osteogenic lineages. Exp Dermatol. 12 (2003), pp. 849-859

C.A. Jahoda, A.J. Reynolds. Hair follicle dermal sheath cells: unsung participants in wound healing. Lancet, 358 (2001), pp. 1445-1448

M.F. Pittenger, A.M. Mackay, S.C. Beck, R.K. Jaiswal, R. Douglas, J.D. Mosca, et al. Multilineage potential of adult human mesenchymal stem cells. Science, 284 (1999), pp. 143-147

S.R. Opalenik, J.M. Davidson. Fibroblast differentiation of bone marrow-derived cells during wound repair. FASEB J, 19 (2005), pp. 1561-1563

N.C. Direkze, S.J. Forbes, M. Brittan, T. Hunt, R. Jeffery, S.L. Preston, et al. Multiple organ engraftment by bone-marrow-derived myofibroblasts and fibroblasts in bone-marrow-transplanted mice. Stem Cells, 21 (2003), pp. 514-520

Friedenstein, A.J., R.K. Chailakhyan, N.V. Latsinik, A.F. Panasyuk, and I.V. Keiliss-Borok. 1974. Stromal cells responsible for transferring the microenvironment of the hemopoietic tissues. Cloning in vitro and retransplantation in vivo. Transplantation. 17: 331-40.

Prockop, D.J. 1997. Marrow stromal cells as stem cells for nonhematopoietic tissues. Science. 276: 71-4.

Uccelli, A., Moretta, L., Pistoia, V. (2008). Mesenchymal stem cells in health and disease. Nat Rev Immunol 8:726-736.

Le Blanc K, Frassoni F, Ball L, Locatelli F, Roelofs H, Lewis I, et al. (2008). Mesenchymal stem cells for treatment of steroid-resistant, severe, acute graft-versus-host disease: a phase II study. Lancet 371:1579-1586.

Sun L, Akiyama K, Zhang H, Yamaza T, Hou Y, Zhao S, et al. (2009). Mesenchymal stem cell transplantation reverses multiorgan dysfunction in systemic lupus erythematosus mice and humans. Stem Cells 27:1421-1432.

Akiyama K, Chen C, Wang D, Xu X, Qu C, Yamaza T, et al. (2012). Mesenchymal-stem-cell-induced immunoregulation involves FAS-ligand-/FAS-mediated T cell apoptosis. Cell Stem Cell 10:544-555.

Xu X, Chen C, Akiyama K, Chai Y, Le AD, Wang Z, et al. (2013). Gingivae contain neural-crest- and mesoderm-derived mesenchymal stem cells. J Dent Res. 92(9):825-832.

Fournier BP, Larjava H, Häkkinen L. (2013) Gingiva as a source of stem cells with therapeutic potential. Stem Cells Dev 22(24): 3157-3177.

Su WR, Zhang QZ, Shi SH, Nguyen AL, Le AD (2011). Human gingivaderived mesenchymal stromal cells attenuate contact hypersensitivity via prostaglandin E(2)-dependent mechanisms. Stem Cells 29:1849-1860.

Tang L, Li N, Xie H, Jin Y (2011). Characterization of mesenchymal stem cells from human normal and hyperplastic gingiva. J Cell Physiol 226:832-842.

Zhang Q, Shi S, Liu Y, Uyanne J, Shi Y, Shi S, et al. (2009). Mesenchymal stem cells derived from human gingiva are capable of immunomodulatory functions and ameliorate inflammation-related tissue destruction in experimental colitis. J Immunol 183:7787-7798.

Zhang Q, Su WR, Shi SH, Wilder-Smith P, Xiang AP, Wong A, et al. (2010). Human gingiva-derived mesenchymal stem cells elicit polarization ofm2 macrophages and enhance cutaneous wound healing. Stem Cells 28:1856-1868.

Garlanda C, Dinarello CA, Mantovani A. The interleukin-1 familiy: back to future. (2013) Immunity. 39(6): 1003-1018.

Muzio M, Polentarutti N, Sironi M, Poli G, De Gioia L, Introna M, et al. (1995) Cloning and Characterization of a New Isoformof the Interleukin 1 Receptor Antagonist. J Exp Med. 182(2): 623-628.

F.L. van de Veerdonk, A.K. Stoeckman, G. Wu, A.N. Boeckermann, T. Azam, M.G. Netea, L.A. Joosten, J.W. van der Meer, R. Hao, V. Kalabokis, C.A. Dinarello. (2012) IL-38 binds to the IL-36 receptor and has biological effects on immune cells similar to IL-36 receptor antagonist. Proc. Natl. Acad. Sci. USA, 109: 3001-3005.

Arend, WP. The balance between IL-1 and IL-1ra in disease. (2002) Cytokine Growth Factor Rev, 13:323-340.

Ishida Y, Kondo T, Kimura A, Matsushima K, Mukaida N. Absence of IL-1 receptor antagonist inmaired wound healing along with aberrant NF-kappaB activation and reciprocal suppression of TGF-beta signal pathway. (2006) J Immunol, 176(9): 5598-5606.

Horai R, Saijo S, Tanioka H, Nakae S, Sudo K, Okahara A, et al. (2000) Development of chronic inflammatory arthropathy resembling rheumatoid arthritis in interleukin 1 receptor antagonist-deficient mice. J Exp Med. 191: 313-320.

M.J. Nicklin, D.E. Hughes, J.L. Barton, J.M. Ure, G.W. Duff. (2000) Arterial inflammation in mice lacking the interleukin 1 receptor antagonist gene. J Exp Med. 191: 303-312.

I. Aksentijevich, S.L. Masters, P.J. Ferguson, P. Dancey, J. Frenkel, A. van Royen-Kerkhoff, R. Laxer, U. Tedgård, E.W. Cowen, T.H. Pham, et al. (2009)An autoinflammatory disease with deficiency of the interleukin-1-receptor antagonist. N Engl J Med. 360: 2426-2437.

S. Reddy, S. Jia, R. Geoffrey, R. Lorier, M. Suchi, U. Broeckel, et al. (2009) An autoinflammatory disease due to homozygous deletion of the IL1RN locus. N Engl J Med. 360: 2438-2444.

Pittenger, M.F. et al. (1999) Multilineage potential of adult human mesenchymal stem cells. Science 284, 143-147.

Phinney, D.G. and Prockop, D.J. (2007) Concise review: mesenchymal stem/multipotent stromal cells: the state of transdifferentiation and modes of tissue repair - current views. Stem Cells 25, 2896-2902.

Medzhitov, R. (2008) Origin and physiological roles of inflammation. Nature 454, 428-435

Luster, A.D. et al. (2005) Immune cell migration in inflammation: present and future therapeutic targets. Nat. Immunol. 6, 1182- 1190

Eming, S.A. et al. (2007) Inflammation in wound repair: molecular and cellular mechanisms. J. Invest. Dermatol. 127, 514-525

Sasaki, M. et al. (2008) Mesenchymal stem cells are recruited into wounded skin and contribute to wound repair by transdifferentiation into multiple skin cell type. J. Immunol. 180, 2581-2587.

Wu, Y. et al. (2007) Mesenchymal stem cells enhance wound healing through differentiation and angiogenesis. Stem Cells 25, 2648-2659.

Chen, L. et al. (2008) Paracrine factors of mesenchymal stem cells recruit macrophages and endothelial lineage cells and enhance wound healing. PLoS ONE 3, e1886.

Gnecchi, M. et al. (2005) Paracrine action accounts for marked protection of ischemic heart by Akt-modified mesenchymal stem cells. Nat. Med. 11, 367-368.

Timmers, L. et al. (2011) Human mesenchymal stem cell-conditioned medium improves cardiac function following myocardial infarction. Stem Cell Res. 6, 206-214.

Shi Y, Su J, Roberts AI, Shou P, Rabson AB, Ren G. How mesenchymal stem cells interact with tissue immune responses. Trends Immunol 2012; 33: 136-143.

Aguilar S, Scotton CJ, McNulty K, Nye E, Stamp G, Laurent G et al. Bone marrow stem cells expressing keratinocyte growth factor via an inducible lentivirus protects against bleomycin-induced pulmonary fibrosis. PLoS One 2009; 4: e8013.

Hung SP, Yang MH, Tseng KF, Lee OK. Hypoxia-induced secretion of TGF-beta 1 in mesenchymal stem cell promotes breast cancer cell progression. Cell Transplant 2012; 22: 1869-1882.

Krampera, M. et al. (2006) Role for interferon-gamma in the immunomodulatory activity of human bone marrow mesenchymal stem cells. Stem Cells 24, 386-398

Sheng, H. et al. (2008) A critical role of IFNgamma in priming MSC-mediated suppression of T cell proliferation through up-regulation of B7-H1. Cell Res. 18, 846-857

Mougiakakos, D. et al. (2011) The impact of inflammatory licensing on heme oxygenase-1-mediated induction of regulatory T cells by human mesenchymal stem cells. Blood 117, 4826-4835

Hübner G, Brauchle M, Smola H, Madlener M, Fässler R, Werner S. (1996) Differential regulation of pro-inflammatory cytokines during wound healing in normal and glucocorticoid-treated mice. Cytokine 8(7): 548-556.

Miller, L. S. et al. MyD88 mediates neutrophil recruitment initiated by IL-1R but not TLR2 activation in immunity against Staphylococcus aureus. (2006) Immunity 24, 79-91.

Dinarello, C. A. Biologic basis for interleukin-1 in disease. (1996) Blood 87, 2095-2147.

Chung, Y.et. al. CriticalregulationofearlyTh17 cell differentiation by interleukin-1 signaling. (2009) Immunity 30, 576-587.

Acosta-Rodriguez, e. v., Napolitani, G., Lanzavecchia, A. & Sallusto, F. Interleukins 1β and 6 but not transforming growth factor-β are essential for the differentiation of interleukin 17-producing human T helper cells. (2007) Nat. Immunol. 8: 942-949.

Greenfeder, S. A. et al. Molecular cloning and characterization of a second subunit of the interleukin 1 receptor complex. (1995) J. Biol. Chem. 270, 13757-13765.

Arend, W. P., Malyak, M., Guthridge, C. J. & Gabay, C. Interleukin-1 receptor antagonist: role in biology. (1998) Annu. Rev. Immunol. 16, 27-55.

Palmer, G., Talabot-Ayer, D., Kaya, G. & Gabay, C. Type I IL-1 receptor mediates IL-1 and intracellular IL-1 receptor antagonist effects in skin inflammation. (2007) J. Invest. Dermatol. 127, 1938-1946.

Tibbetts, M. D., Zheng, L. & Lenardo, M. J. The death effector domain protein family: regulators of cellular homeostasis. (2003) Nature Immunol. 4, 404-409.

Brint E, O'Callaghan G, Houston A. (2013) Life in the fas lane: differential outcomes of fas signaling. Cell Mol Life Sci, 70:4085-4099.

Trauth BC, Klas C, Peters AM, Matzku S, Moller P, Falk W, Debatin KM, Krammer PH (1989) Monoclonal antibody- mediated tumor regression by induction of apoptosis. Science 245:301-305

Yonehara S, Ishii A, Yonehara M (1989) A cell-killing mono- clonal antibody (anti-Fas) to a cell surface antigen co-downregulated with the receptor of tumor necrosis factor. J Exp Med 169:1747-1756

Locksley RM, Killeen N, Lenardo MJ (2001) The TNF and TNF receptor superfamilies: integrating mammalian biology. Cell 104:487-501

Scott FL, Stec B, Pop C, Dobaczewska MK, Lee JJ, Monosov E, Robinson H, Salvesen GS, Schwarzenbacher R, Riedl SJ (2009) The Fas-FADD death domain complex structure unravels signaling by receptor clustering. Nature 457:1019-1022

Scaffidi C, Fulda S, Srinivasan A, Friesen C, Li F, Tomaselli KJ, Debatin KM, Krammer PH, Peter ME (1998) Two CD95 (APO- 1/Fas) signaling pathways. EMBO J 17:1675-1687

Kovacs B, Tsokos GC (1995) Cross-linking of the Fas/APO-1 antigen suppresses the CD3-mediated signal transduction events in human T lymphocytes. J Immunol 155:5543-5549

Alderson MR, Armitage RJ, Maraskovsky E, Tough TW, Roux E, Schooley K, Ramsdell F, Lynch DH (1993) Fas transduces activation signals in normal human T lymphocytes. J Exp Med 178:2231-2235

Freiberg RA, Spencer DM, Choate KA, Duh HJ, Schreiber SL, Crabtree GR, Khavari PA (1997) Fas signal transduction triggers either proliferation or apoptosis in human fibroblasts. J Invest Dermatol 108:215-219

Chen L, Park SM, Tumanov AV, Hau A, Sawada K, Feig C, Turner JR, Fu YX, Romero IL, Lengyel E, Peter ME (2010) CD95 promotes tumour growth. Nature 465:492-496

Desbarats J, Newell MK (2000) Fas engagement accelerates liver regeneration after partial hepatectomy. Nat Med 6:920-923

Reinehr R, Sommerfeld A, Haussinger D (2008) CD95 ligand is a proliferative and antiapoptotic signal in quiescent hepatic stel late cells. Gastroenterology 134:1494-1506

Lambert C, Landau AM, Desbarats J (2003) Fas-beyond death: a regenerative role for Fas in the nervous system. Apoptosis 8:551-562

Barnhart BC, Legembre P, Pietras E, Bubici C, Franzoso G, Peter ME (2004) CD95 ligand induces motility and invasiveness of apoptosis-resistant tumor cells. EMBO J 22:22

Li H, Fan X, Stoicov C, Liu JH, Zubair S, Tsai E, Ste Marie R, Wang TC, Lyle S, Kurt-Jones E, Houghton J (2009) Human and mouse colon cancer utilizes CD95 signaling for local growth and metastatic spread to liver. Gastroenterology 137:934-944

Trauzold A, Roder C, Sipos B, Karsten K, Arlt A, Jiang P, Mar- tin-Subero JI, Siegmund D, Muerkoster S, Pagerols-Raluy L, Siebert R, Wajant H, Kalthoff H (2005) CD95 and TRAF2 pro- mote invasiveness of pancreatic cancer cells. Faseb J 19:620-622

Letellier E, Kumar S, Sancho-Martinez I, Krauth S, Funke- Kaiser A, Laudenklos S, et al. (2010) CD95-ligand on peripheral myeloid cells activates Syk kinase to trigger their recruitment to the inflammatory site. Immunity 32:240-252

Zhang Y, Liu Q, Zhang M, Yu Y, Liu X, Cao X (2009) Fas signal promotes lung cancer growth by recruiting myeloid-derived suppressor cells via cancer cell-derived PGE2. J Immunol 182:3801-3808

Palao G, Santiago B, Galindo MA, Rullas JN, Alcami J, Ramirez JC, Pablos JL (2006) Fas activation of a proinflammatory program in rheumatoid synoviocytes and its regulation by FLIP and caspase 8 signaling. Arthritis Rheum 54:1473-1481

Ma Y, Liu H, Tu-Rapp H, Thiesen HJ, Ibrahim SM, Cole SM, Pope RM (2004) Fas ligation on macrophages enhances IL- 1R1 -Toll-like receptor 4 signaling and promotes chronic inflammation. Nat Immunol 5:380-387

Eramo A, Sargiacomo M, Ricci-Vitiani L, Todaro M, Stassi G, et al. (2004) CD95 death-inducing signaling complex formation and internalization occur in lipid rafts of type I and type II cells. Eur J Immunol 34: 1930-1940

Gajate C, Mollinedo F (2001) The antitumor ether lipid ET-18-OCH(3) induces apoptosis through translocation and capping of Fas/CD95 into membrane rafts in human leukemic cells. Blood 98: 3860-3863.

Zhu XD, Zhuang Y, Ben JJ, Qian LL, Huang HP, et al. (2011) Caveolae-dependent endocytosis is required for class A macrophage scavenger receptor-mediated apoptosis in macrophages. J Biol Chem 286: 8231-8239.

Guo YH, Hernandez I, Isermann B, Kang TB, Medved L, et al. (2009) Caveolin-1-dependent apoptosis induced by fibrin degradation products. Blood 113: 4431-4439.

Parton RG, del Pozo MA. Caveolae as plasma membrane sensors, protectors and organizers. (2013) Nat Rev Mol Cell Biol, 14(2): 98-112.

Cao H, Courchesne WE, Mastick CC. (2002) A phosphotyrosine-dependent protein interaction screen reveals a role for phosphorylation of caveolin-1 on tyrosine 14: recruitment of C-terminal Src kinase. J Biol Chem 277: 8771-8774.

del Pozo, M. A. et al. Phospho-caveolin-1 mediates integrin-regulated membrane domain internalization. (2005) Nat Cell Biol, 7: 901-908.

Wrana JL, Attisano L, Cárcamo J, Zentella A, Doody J, Laiho M et al. TGF beta signals through a heteromeric protein kinase receptor complex. (1992) Cell. 71(6): 1003-1014.

Cahill DP, Lengauer C, Yu J, Riggins GJ, Willson JK, Markowitz SD, et al. Mutations of mitotic checkpoint genes in human cancers. (1998) Nature. 392(6673): 300-303.

Wang Y, Chen X, Cao W, Shi Y. Plasticity of mesenchymal stem cells in immunomodulation: pathological and therapeutic implications. (2014) Nat Immunol. 15(11): 1009-1016.

Valadi H, Ekström K, Bossios A, Sjöstrand M, Lee JJ, Lötvall JO. Exosome-mediated transfer of mRNAs and microRNAs is a novel mechanism of genetic exchange between cells. (2007) Nat Cell Biol. 9: 654-659.

Mendez-Ferrer, S. et al. Mesenchymal and haematopoietic stem cells form a unique bone marrow niche. Nature 466, 829-834 (2010).

Sutton RB, Fasshauer D, Jahn R, Brunger AT (1998). "Crystal structure of a SNARE complex involved in synaptic exocytosis at 2.4 Å resolution". Nature 395 (6700): 347-353.

The present invention relates to the embodiments set out in the appended set of claims.

This disclosure relates to a stem cell treatment. This disclosure also relates to an IL-1RA treatment. This disclosure also relates to a composition comprising stem cells and to the preparation of this composition. This disclosure also relates to a composition comprising IL-1RA and to the preparation of this composition. This disclosure further relates to a wound treatment using stem cells and/or IL-1RA. This disclosure also further relates to a treatment to reduce scar formation by using a stem cell and/or IL-1RA. A composition comprising a stem cell that may treat a wound. Preferably, the stem cells compositions are prepared from multipotent or pluripotent stem cells, and not from totipotent stem cells. More preferably, the stem cell compositions are prepared from multipotent stem cells, and not from pluripotent or totipotent stem cells.

As described and exemplified herein, certain stem cells contain intracellular IL-1RA, and can release the intracellular IL-1RA in response to stimuli (e.g., pro-inflammatory mediators) that are present at the site of a would. Thus, IL-1RA can be administered to a subject in need thereof by administering stem cells that contain intracellular IL-1RA to the subject. In some aspects, the disclosure relates to a composition that may comprise IL-1RA in which the composition contains a stem cell with intracellular IL-1RA.

The composition may comprise IL-1 RA, which may treat a wound. This composition may improve wound healing. The composition may also comprise IL-1 RA substitute, which may treat a wound. An example of an IL-1 RA substitute may be anakinra, which is a recombinant, nonglycosylated form of the human IL-1 RA. Anakinra differs from native human IL-1 RA in that it has the addition of a single methionine residue at its amino terminus. It may be produced by recombinant DNA technology using an *E coli* bacterial expression system. The composition may comprise IL-1RA substitute with a concentration, for example, in the range of 1 mg per mL to 1,000 mg per mL of the composition; or 10 mg per mL to 200 mg per mL of the composition. The composition may comprise IL-1RA substitute with a concentration, for example, in the range of 1 mg per gram to 1,000 mg per gram of the composition; or 10 mg per gram to 200 mg per gram of the composition.

The composition may comprise a stem cell and may reduce scar formation when the composition is administered to treat a wound (e.g. applied to a wound). The composition may also comprise IL-1RA and may reduce scar formation when the composition is administered to treat a wound (e.g., applied to a wound).

The composition may comprise stem cells, for example, in the form of a cell culture. The composition may be a drug or a biologic formulation. The stem cell may be any stem cell. The stem cell may comprise gingiva derived mesenchymal stem cell (GMSC).

The wound may be any injury to any living tissue. An example of tissue may be skin. Examples of injury may be caused by trauma, such as a cut, blow, or other impact, including accidental and surgical reasons. The living tissue may be a living tissue of a mammal. The mammal may be a human. The mammal may be a non-human animal.

The treatment may comprise administering one or more compositions using any suitable methods, such as systemic infusion, localized application (including topical application), or other suitable means of formulation and delivery.

The preparation of a composition comprising a stem cell may comprise obtaining a tissue, separating the tissue into cells, identifying and/or isolating stem cells (e.g., by sorting), and preparing a composition comprising the stem cells.

Other examples are as follows.

A composition may comprise an IL-1RA substitute and at least one human stem cell that expresses IL-1 RA. This composition may comprise an effective amount of the IL-1 RA substitute such that the composition is effective in a treatment of a wound. The composition may comprise an effective amount of the IL-1 RA substitute such that the composition is effective in reducing scar formation when the composition is applied to a wound. The composition may comprise an effective amount of human stem cells that are effective in a treatment of a wound. The composition may comprise an effective amount of human stem cells such that the composition is effective in reducing scar formation when the composition is applied to a wound. The composition may comprise an effective amount of the IL-1 RA substitute and an effective amount of human stem cells such that the composition is effective in a treatment of a wound. The composition may comprise the IL-1 RA substitute and an effective amount of human stem cells such that the composition is effective in reducing scar formation when the composition is applied to a wound. The stem cell may comprise a human mesenchymal stem cell. The human stem cell may comprise a human bone marrow-derived mesenchymal stem cell, a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human gingiva-derived mesenchymal stem cell, a human periosteum-derived stem cell, a human salivary gland-derived stem cell, or a combination thereof. The human stem cell may comprise a human gingiva-derived mesenchymal stem cell. The IL-1RA substitute may be anakinra.

A method of treating a human subject may comprise administering an effective amount of a composition comprising human IL-1 RA to the human subject, and thereby treating a wound of the human subject. The human IL-1 RA may be derived from at least one human stem cell. The composition may comprise an effective amount of the human IL-1 RA such that the composition is effective in the treatment of the wound. The human stem cell may comprise a mesenchymal stem cell. The human stem cell may comprise a human bone marrow-derived mesenchymal stem cell, a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human gingiva-derived mesenchymal stem cell, a human periosteum-derived stem cell, a human salivary gland-derived stem cell, or a combination thereof. The human stem cell may comprise a human gingiva-derived mesenchymal stem cell.

A method of treating a human subject may comprise administering an effective amount of a composition comprising an IL-1 RA substitute to the human subject and thereby treating a wound of the human subject. The composition may comprise an effective amount of the IL-1 RA substitute such that the composition is effective in the treatment of the wound. The composition may comprise an effective amount of the IL-1 RA substitute such that the composition is effective in reducing scar formation when the composition is applied to the wound. The IL-1 RA substitute may be anakinra.

A method of treating a human subject may comprise administering an effective amount of a composition comprising an IL-1RA substitute and at least one human stem cell that expresses IL-1 RA to the human subject, and thereby treating a wound of the human subject. The composition may comprise an effective amount of the IL-1 RA substitute such that the composition is effective in the treatment of the wound. The composition may comprise an effective amount of the IL-1 RA substitute such that the composition is effective in reducing scar formation when the composition is applied to the wound. The composition may comprise an effective amount of human stem cells that are effective in the treatment of the wound. The composition may comprise an effective amount of human stem cells such that the composition is effective in reducing scar formation when the composition is applied to the wound. The composition may comprise an effective amount of the IL-1 RA substitute and an effective amount of the human stem cells such that the composition is effective in the treatment of the wound. The composition may comprise the IL-1 RA substitute and an effective amount of human stem cells such that the composition is effective in reducing scar formation when the composition is applied to the wound. The human stem cell may comprise a human mesenchymal stem cell. The human stem cell may comprise a human bone marrow-derived mesenchymal stem cell, a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human gingiva-derived mesenchymal stem cell, a human periosteum-derived stem cell, a human salivary gland-derived stem cell, or a combination thereof. The human stem cell may comprise a human gingiva-derived mesenchymal stem cell. The IL-1 RA substitute may be anakinra.

A composition may comprise human IL-1RA. The human IL-1RA may be derived from at least one human stem cell. The composition may comprise an effective amount of the human IL-1 RA such that the composition is effective in a treatment of a wound. The composition may comprise an effective amount of the human IL-1 RA such that the composition is effective in reducing scar formation when the composition is applied to a wound. The human stem cell may comprise a mesenchymal stem cell. The human stem cell may comprise a human bone marrow-derived mesenchymal stem cell, a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human gingiva-derived mesenchymal stem cell, a human periosteum-derived stem cell, a human salivary gland-derived stem cell, or a combination thereof. The human stem cell may comprise a human gingiva-derived mesenchymal stem cell.

The composition may further comprise a human stem cell. The human stem cell may comprise a human mesenchymal stem cell. The human stem cell may comprise a human bone marrow-derived mesenchymal stem cell, a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human gingiva-derived mesenchymal stem cell, a human periosteum-derived stem cell, a human salivary gland-derived stem cell, or a combination thereof. The human stem cell may comprise a human gingiva-derived mesenchymal stem cell.

The composition may further comprise a stem cell culture. The stem cell culture may comprise a human mesenchymal stem cell. The stem cell culture may comprise a human bone marrow-derived mesenchymal stem cell, a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human gingiva-derived mesenchymal stem cell, a human periosteum-derived stem cell, a human salivary gland-derived stem cell, or a combination thereof. The stem cell culture may comprise a human gingiva-derived mesenchymal stem cell.

The wound may be any injury to any living tissue of a mammal. The tissue may be skin. The injury may be caused by a cut or blow. The mammal may be a human. The mammal may be a non-human animal.

Any combination of features disclosed above may be possible and thereby within the scope of this disclosure. For example, any combination of above products (e.g. compositions) and methods (e.g. methods of treatment) is within the scope of this disclosure.

These, as well as other components, steps, features, objects, benefits, and advantages, will now become clear from a review of the following detailed description, the accompanying drawings, and the claims.

When the same numeral appears in different drawings, it refers to the same or like components or steps.

The figures show:

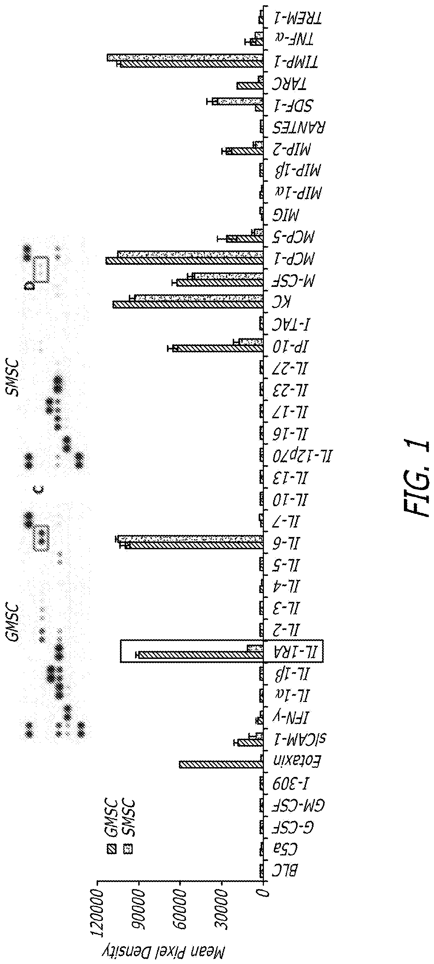

**FIG. 1****.** Cytokine array data showed that GMSCs expressed significant higher level of several cytokines including IL-1RA than SMSCs.

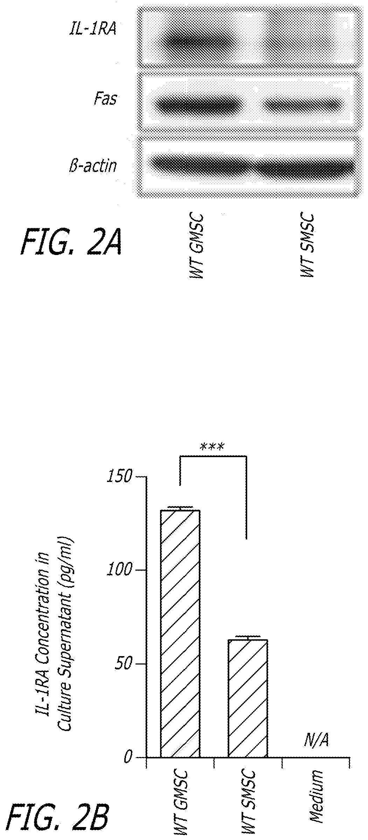

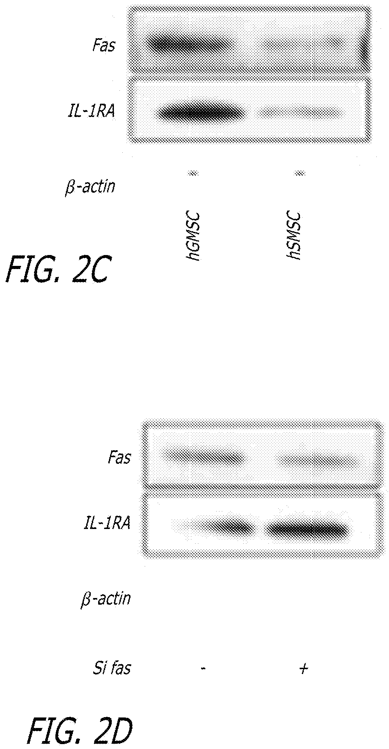

**FIG. 2****.** (A) Western blotting showed SMSCs express less IL-1RA and FAS compared with GMSCs in wild-type mouse. (B) ELISA showed GMSCs released higher level of IL-1RA in culture supernatant compared with SMSCs. (C) Western blotting data showed that human GMSCs have the same trend as the mouse GMSCs, which expressed a higher FAS and IL-1RA in cytoplasm. (D) Western blotting showed that when use fas siRNA to block the FAS expression in human GMSCs, the IL-1RA level in human GMSCs will increase just as the observation in mouse GMSCs.

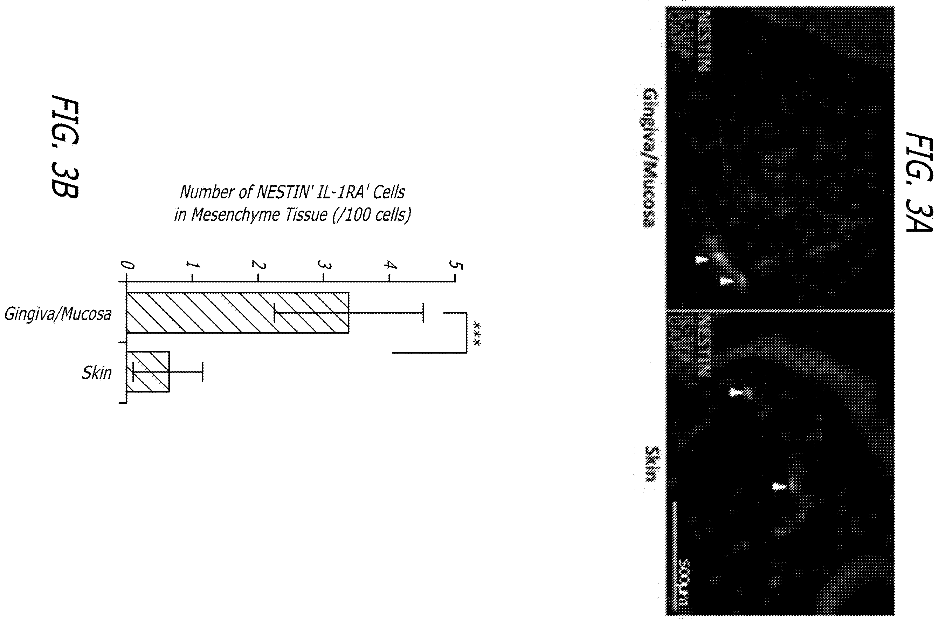

**FIG. 3.** (A) Immunofluorescence staining showed NESTIN+ mesenchymal stem cells were positive with IL-1 RA expression in gingiva while not in skin. (B) Statistical analysis showed that in gingiva/mucosa, there were significantly high number of IL-1RA+ and NESTIN+ mesenchymal stem cells when compared with skin.

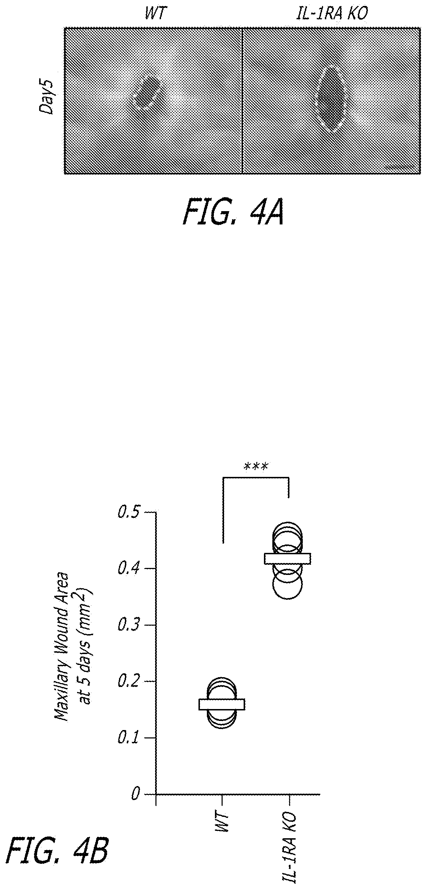

**FIG. 4.** (A) Wound (1.5mm in diameter) healing delayed in IL-1RA KO mice at day 5. (B)Statistical analysis showed that the wound size was significantly larger in the IL-1RA KO mice than in the wild type control.

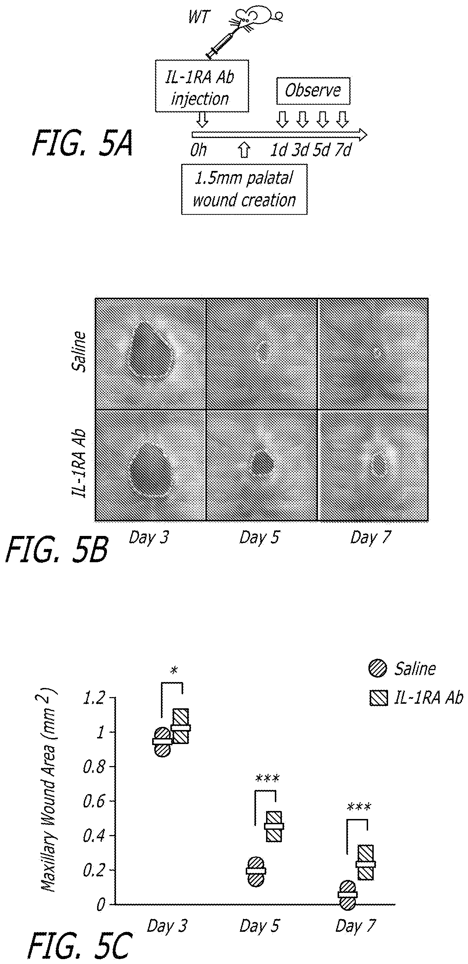

**FIG. 5.** (A) Schematic diagram of using IL-1 RA neutralizing antibody to study the mucosa wound healing. (B) Pretreatment with IL-1 RA neutralizing antibody compromised the mucosa wound healing in WT mice in multiple time points. (C) Statistical analysis showed that during each time course, the IL-1 RA neutralizing antibody significantly delayed the wound healing process in wild type mice.

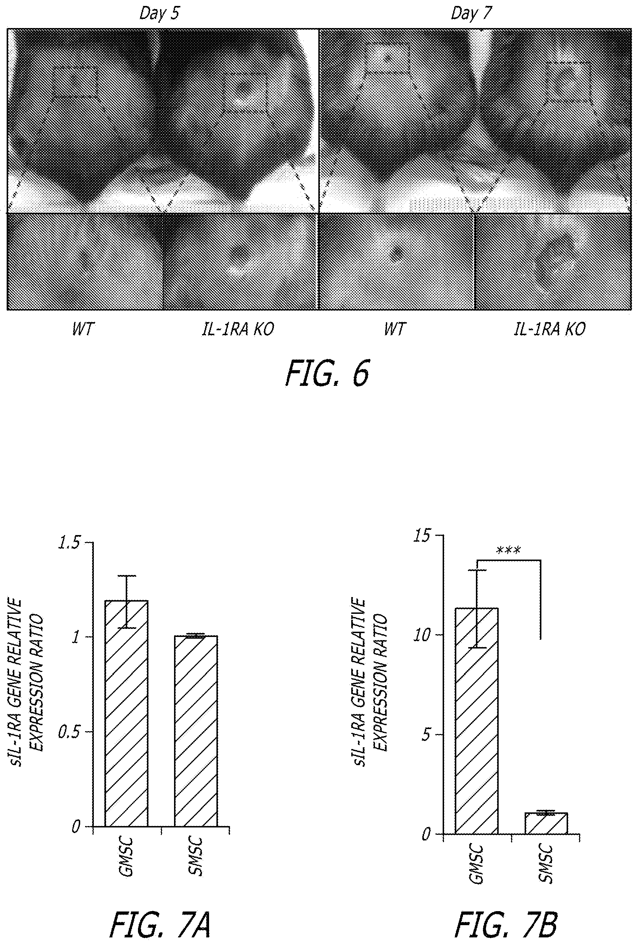

**FIG. 6****.** Skin wound healed much faster in the WT compared with the IL-1RA KO mice at multiple time point.
**FIG. 7.** (A) GMSCs and SMSCs had same mRNA expression level of soluble IL-1RA. (B) GMSCs had a significantly higher mRNA expression level of intracellular IL-1 RA when compared with SMSCs.

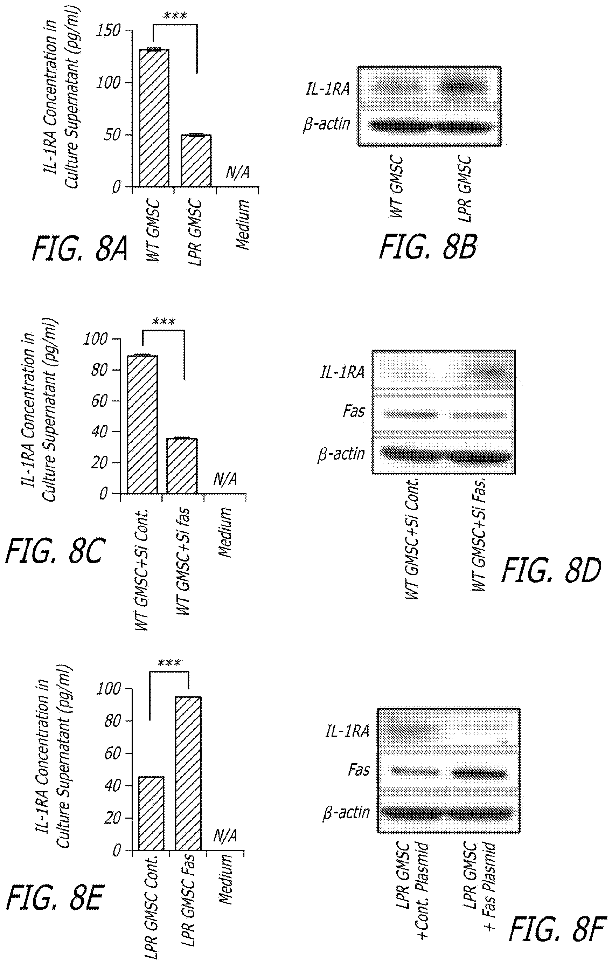

**FIG. 8.** (A) WT GMSCs secreted a higher level of IL-1RA in culture supernatant than Ipr GMSCs. (B) Western blotting showed that Ipr GMSCs remained more IL-1RA in cytoplasm. (C and D) When WT GMSCs with fas gene knock down, the IL-1 RA was blocked in the cell with less secreted into the culture supernatant. (E and F) When Ipr GMSCs over expressed FAS, the IL-1RA secretion was enhanced with less remained in cells.

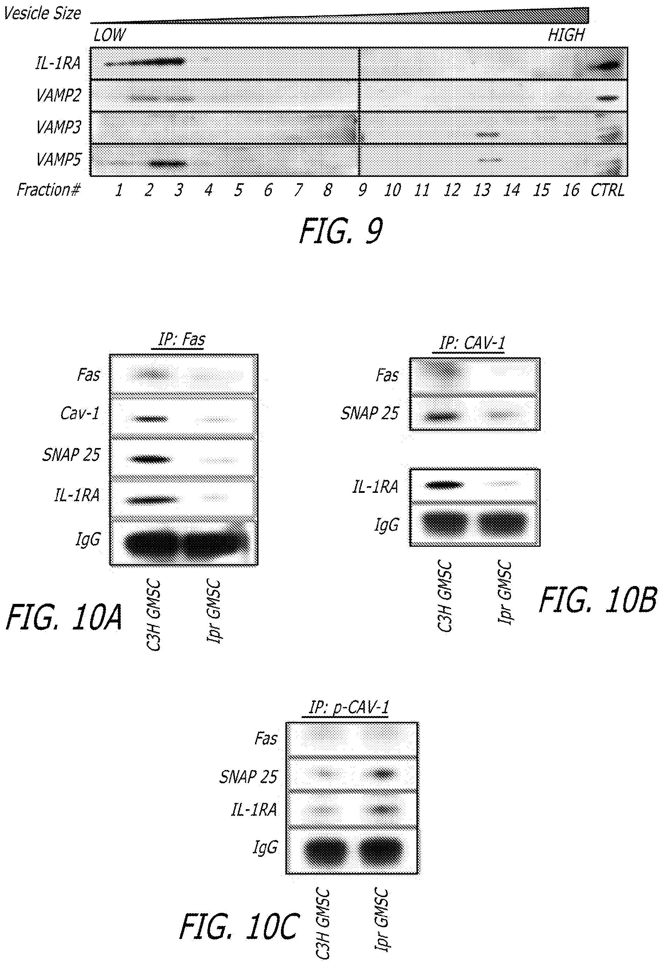

**FIG. 9****.** Sucrose gradient centrifuge showed that IL-1 RA was contained in the vesicle in very small size. The vesicle membrane associated protein 5 (VAMP5) showed a overlapping distribution with IL-1 RA.
**FIG. 10.** (A-C) Immunoprecipitation experiment showed that phosphorylated CAVEOLIN-1 had more binding to SNAP25 and IL-1RA, inhibiting the IL-1RA from release.

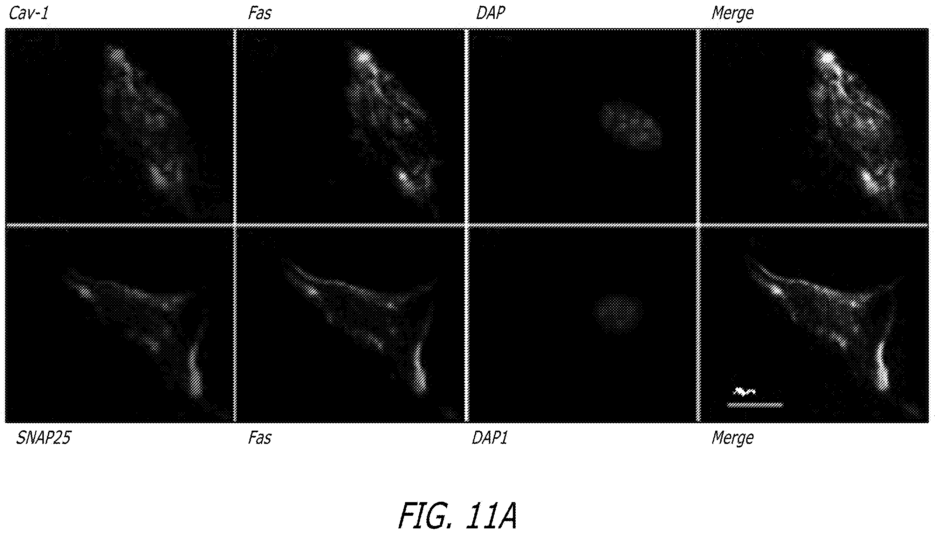

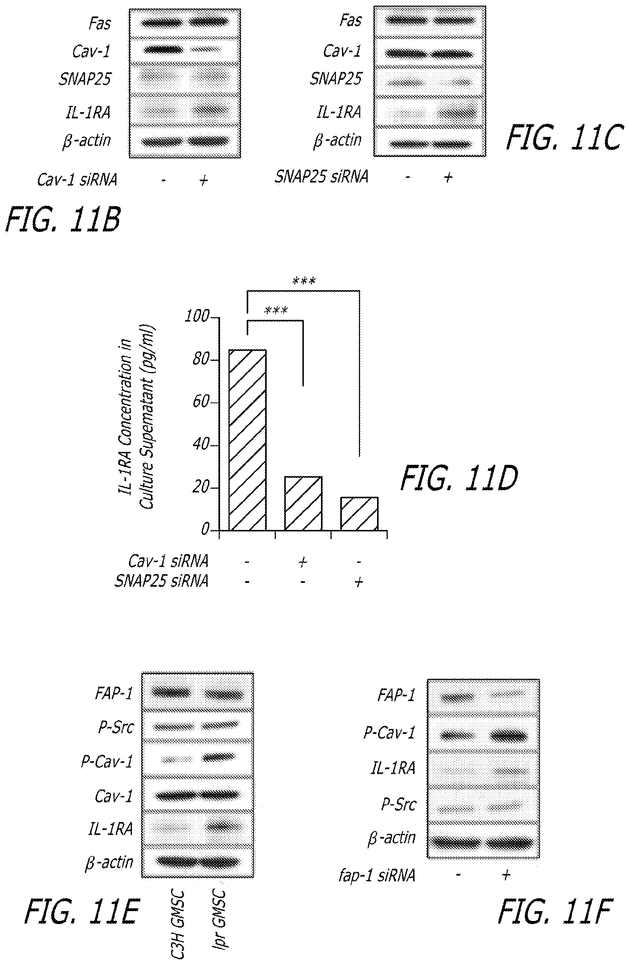

**FIG. 11****.** (A) Immunofluorescence staining showed co-localization of FAS, CAV-1 and SNAP25 on WT GMSCs membrane. (B-D) Knock down cav-1 and snap25 gene by siRNA showed the impaired release of IL-1RA into culture supernatant. (E) Sucrose gradient centrifuge showed that IL-1 RA was contained in the vesicle of small size. (E and F) Western blotting showed that Ipr GMSCs had a higher level of phospho-CAVEOLIN-1, which was due to the FAS expression maintained the proper function of FAP-1. When knock down the fap-1 gene by siRNA, WT GMSCs also showed a higher phospho-CAVEOLIN-1 level.

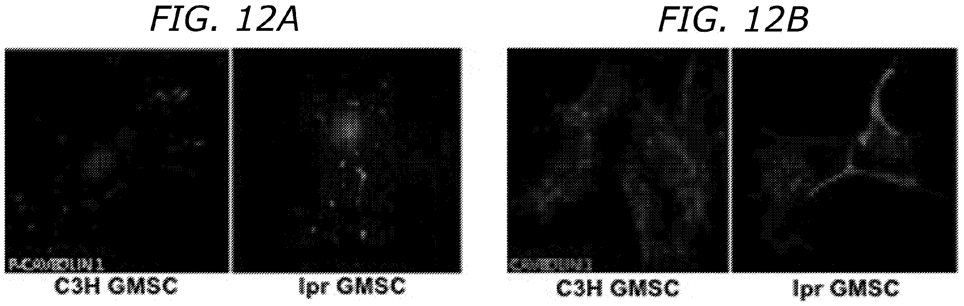

**FIG. 12.** (A) Immunofluorescence staining showed Ipr GMSCs expressed higher level of phospho-CAVEOLIN-1. (B) Immunofluorescence staining showed alter the caveolae distribution in Ipr GMSCs.

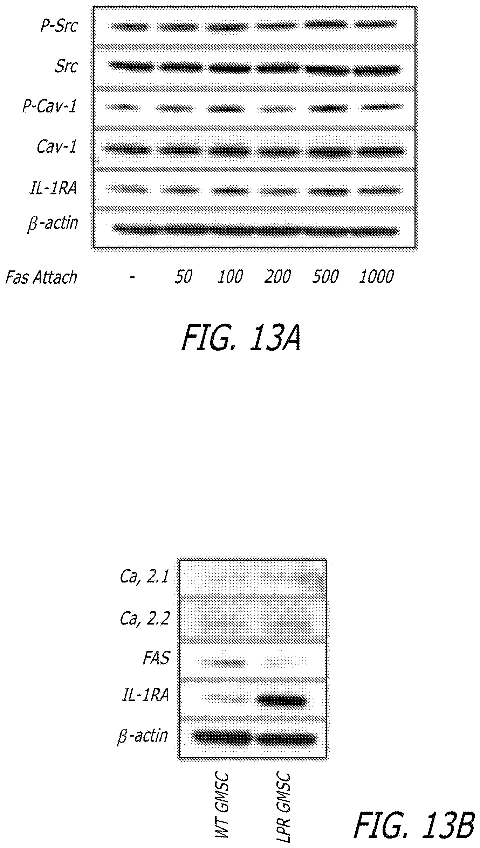

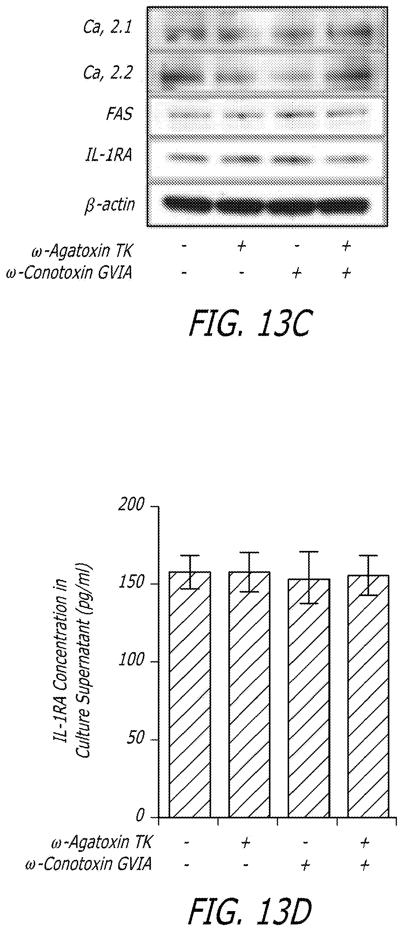

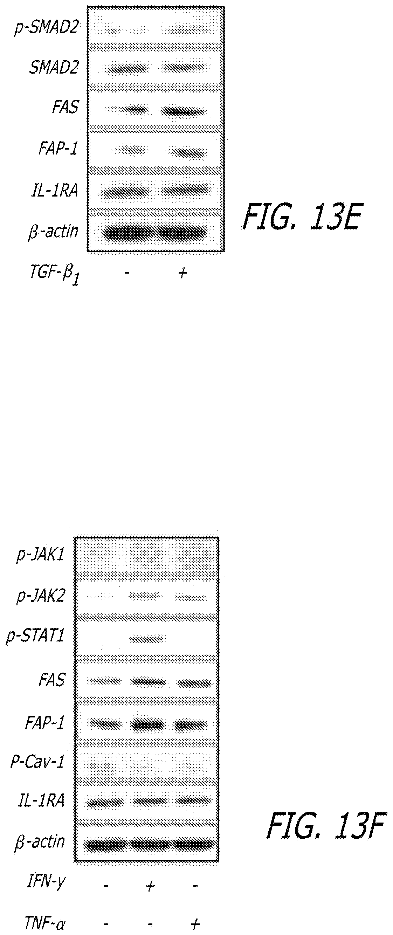

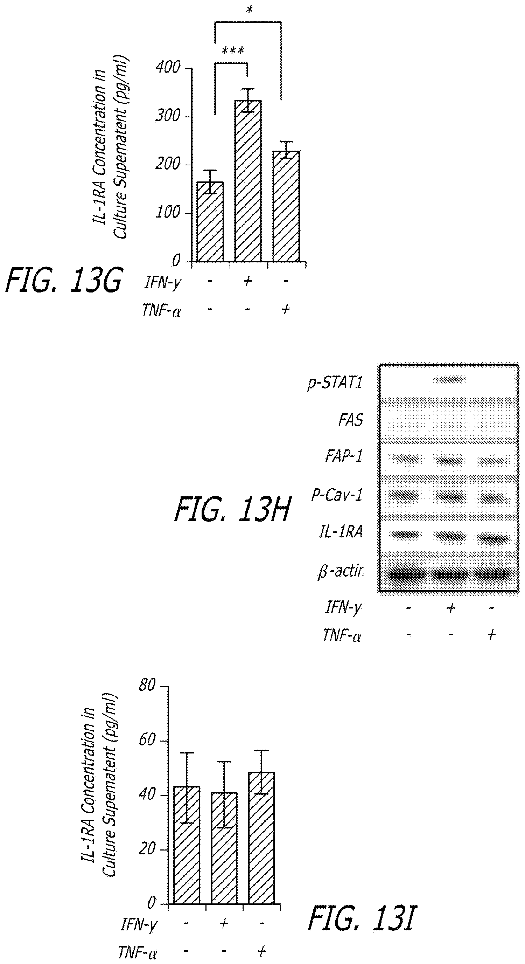

**FIG. 13****.** (A) When using the FAS neutralizing antibody to trigger FAS signaling, WT GMSCs also showed no difference in IL-1RA release. (B) Both WT and Ipr GMSCs expressed the calcium channel protein Cav 2.1 and 2.2 which are responsible for Ca+ controlled vesicle release. (C and D) When using chemical to block both of the calcium channel protein, WT GMSCs showed no difference in IL-1RA release. (E) When using TGF-β1 to stimulate GMSCs, the cells expressed a higher level of FAS and FAP-1. (F) Western blotting showed that after IFN-γ stimulation of the WT GMSCs, the cells showed a decreased level of phospho-CAVEOLIN1, which would further activate the secretory machinery system on cell membrane to release IL-1 RA. (G) The ELISA data confirmed that when stimulated with IFN-γ, WT GMSCs had a higher IL-1RA concentration in culture supernatant. (H) When Ipr GMSCs were treated with IFN-γ, the phospho-CAVEOLIN1 level remained no change due to their lack of functional FAS on the membrane. (I) ELISA data showed that there was no significant concentration change of IL-1 RA found in Ipr GMSCs culture supernatant.

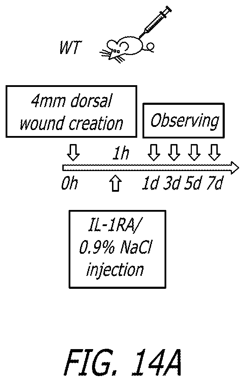

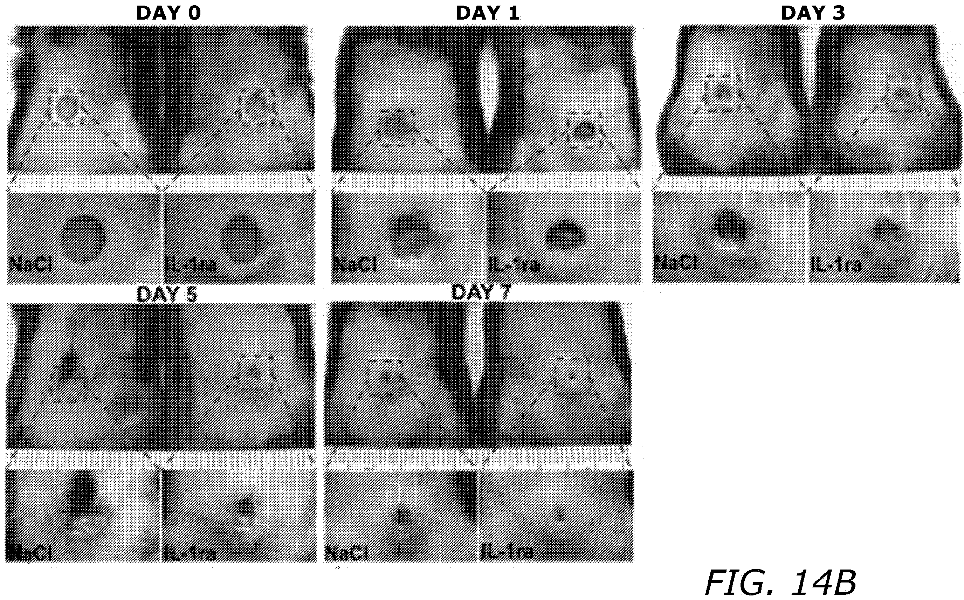

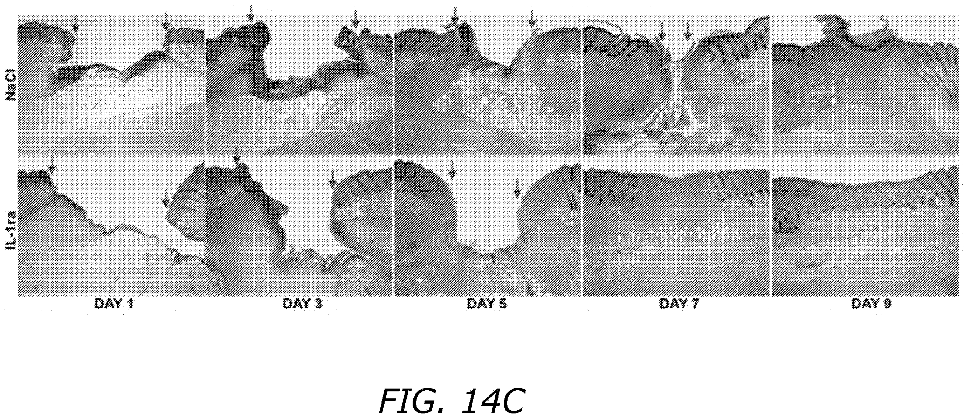

**FIG. 14**. (A) Schematic diagram of using IL-1 RA substitute to rescue the skin wound healing. (B) Skin wound healed much faster in the IL-1RA treated group compared with the saline treated control at multiple time point. (C) H&E staining showed that less inflammatory cells infiltration and better collagen deposition were observed in the IL-1RA treatment group.

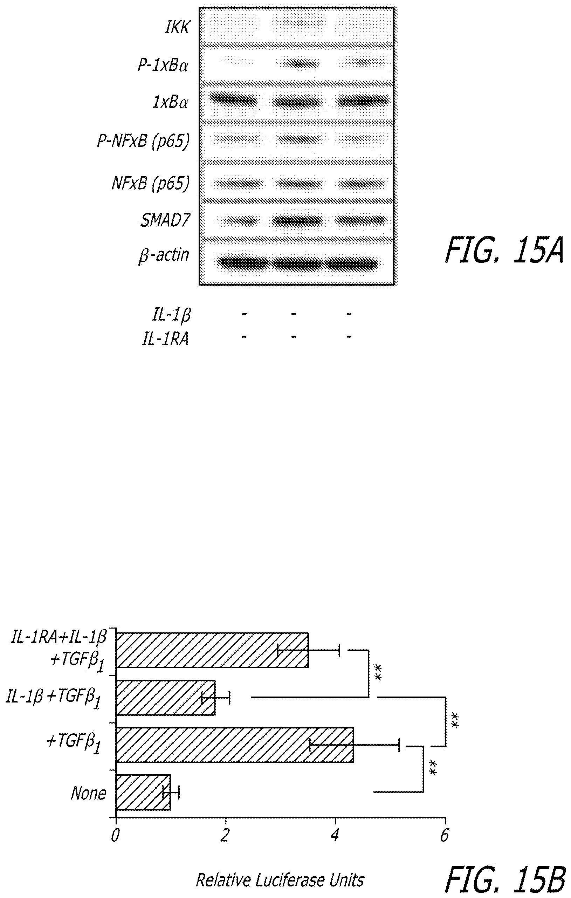

**FIG. 15**. (A) SMSCs when treated with IL-1β had a elevated NF-κB signaling pathway leading to SMAD7 production, which inhibited the TGF-β pathway, while the IL-1RA can antagonize the IL-1β function. (B) TGF-β1 reaction plasmid showed the the IL-1β largely inhibited the TGF-β1 effect on the SMSCs, However IL-1RA could significantly rescue the inhibition.

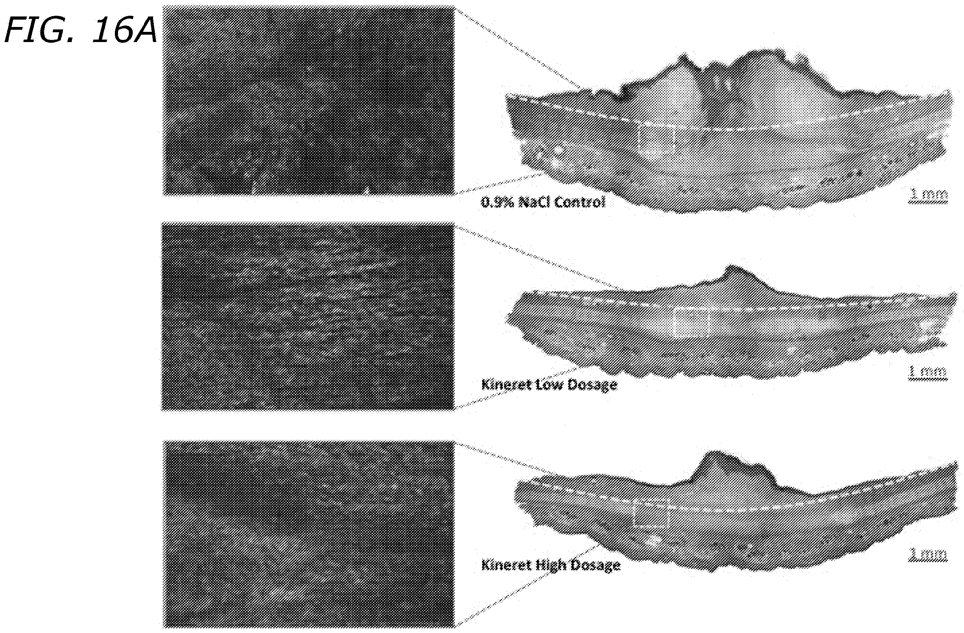

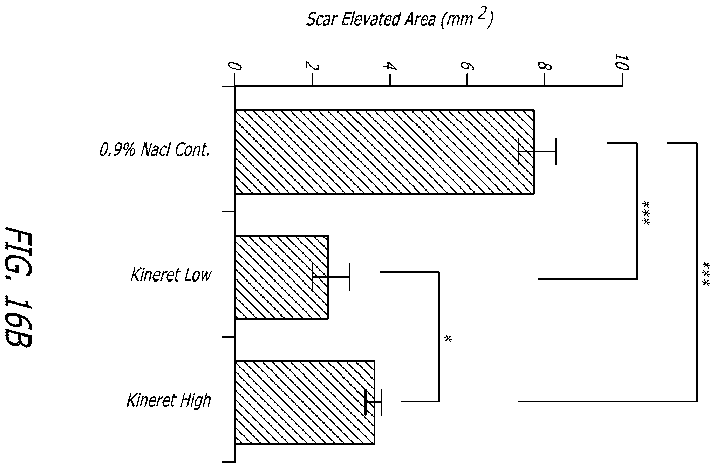

**FIG. 16**. (A) Less scar area and better aligned the collagen deposition were observed in the group of low dosage IL-1 RA treated rabbit ear wound model. Although high dosage of IL-1RA treatment slightly compromised this effect, it still showed significantly better treatment effect when compared with the saline treated control group. (B) Statistical analysis showed that during each time course, the IL-1 RA substitute significantly reduced the scar formation in rabbit ear model.

Illustrative examples are now discussed. Certain details which may be apparent to a person of ordinary skill in the art or unnecessary may have been omitted from this disclosure to provide for a clear and concise disclosure.

The following acronyms and abbreviations were used in this disclosure.
BMMSC: Bone marrow derived mesenchymal stem cells.
Con or Cont: Control.
Co-cul: Co-culture.
ELISA: Enzyme-linked immunosorbent assay technique.
Fas-L: Fas ligand.
GMSC: Gingiva derived mesenchymal stem cell.
g: Gram
IL: Interleukin.
IL-1: interleukin-1.
IL-10: interleukin 10.
IL-1RA: interleukin-1 receptor antagonist.
IFN-γ: interferon-γ.
kg: Kilogram.
mm: Millimeter.
mL: Milliliter.
MSC: Mesenchymal stem cell.
PBS: Phosphate-buffered saline.
PBMC: Peripheral blood mononuclear cells.
PCR: Polymerase chain reaction.
PD: Population doublings
RANKL: Receptor activator of nuclear factor κB ligand.
RT-PCT: Reverse transcription polymerase chain reaction.
SD: Standard deviation.
SMSC: Skin derived mesenchymal stem cell.
TNF: Tumor necrosis factor.
TUNEL: Terminal deoxynucleotidyl transferase-mediated UTP nick-end labeling.
µg: Microgram.
µm: Micrometer.

This disclosure in general relates to a treatment. This disclosure relates to a stem cell treatment. This disclosure also relates to an IL-1RA treatment. This disclosure also relates to a composition comprising stem cells and to methods for the preparation of this composition. This disclosure also relates to a composition comprising IL-1 RA and to methods for the preparation of this composition. This disclosure further relates to a wound treatment using stem cells and/or IL-1 RA. This disclosure also further relates to a treatment to reduce scar formation by using a stem cell and/or IL-1 RA.

The treatment (i.e. method) includes, but not limited to, diagnosis, therapy, cure, healing, mitigation, or prevention of a wound, and/or cosmetic treatment of a mammal. Exemplary methods for treatment (e.g., administering the compositions disclosed herein) include systemic injection or infusion, localized application or injection (including topical application), or other suitable means of formulation and delivery.

This disclosure particularly relates to GMSCs that, when challenged by wound induced inflammatory cytokines, may increase their FAS expression leading to an elevated release of anti-inflammatory peptide(s) (e.g, via microvesicle mediated release) to counterbalance the local inflammatory environment, and thereby may improve wound healing and/or reduce scar formation.

The wound may be any injury to any living tissue. An example of tissue may be skin. Examples of injury may be caused by trauma, such as a cut, blow, or other impact, including accidental and surgical reasons. The living tissue may be a living tissue of a mammal.

The mammal may be a human. The mammal may be a non-human animal. For example, the mammal may be a non-human primate, a horse, a sheep, a cattle, a hog, a dog, a cat, and a goat.

This disclosure relates to a composition. The composition may be a cell culture. The composition may be a drug or a biologic formulation. The composition may be used in the treatment of a wound. An exemplary composition may comprise a stem cell, IL-1RA, an IL-1RA substitute, a stem cell and IL-1RA, a stem cell and an IL-1 RA substitute, a stem cell and IL-1 RA and an IL-1 RA substitute, or a combination thereof. The IL-1 RA can be intracellular IL-1 RA present in a stem cell. Herein, it is preferred that the compositions are prepared from multipotent or pluripotent stem cells, and not from totipotent stem cells. More preferably, the compositions are prepared from multipotent stem cells, and not from pluripotent or totipotent stem cells.

Stem cells, which are suitable for preparation of the exemplary composition, may be any stem cell of any mammal. For example, the stem cells may be stem cells of a mammal that undergoes the treatment (i.e. autologous stem cell treatment). Or, the stem cells may be stem cells of a mammal other than the mammal that undergoes the treatment (i.e. allogeneic stem cell treatment).

Examples of the stem cells may be embryonic stem cells, fetal stem cells, adult stem cells, amniotic stem cells, cord blood stem cells, induced pluripotent stem cells, or combinations thereof.

An example of the stem cells, that are useful in the preparation of the composition, may comprise a mesenchymal stem cell. Examples of mesenchymal stem cells may comprise bone marrow-derived mesenchymal cells, dental pulp stem cells, stem cells from human exfoliated deciduous teeth, periodontal ligament stem cells, dental follicle stem cells, tooth germ progenitor cells, stem cells from the apical papilla, oral epithelial progenitor/stem cells, gingiva-derived mesenchymal stem cells, periosteum-derived stem cells, salivary gland-derived stem cells, adipose derived mesenchymal stem cells, and combinations thereof. For example, the stem cells may be cultured mesenchymal stem cells, uncultured gingiva-derived mesenchymal stem cells, dental pulp stem cells, bone-marrow-derived stem cells, and combinations thereof.

One example of the stem cells, that is useful in the preparation of the composition, may comprise a bone marrow derived mesenchymal stem cell (BMMSC).

Another example of the stem cells, that is useful in the preparation of the composition, may comprise a gingiva derived mesenchymal stem cell (GMSC). The stem cells may be the isolated GMSCs. For isolation of the GMSCs, for example, see Le et al. in a United States patent application publication, entitled "Gingiva Derived Stem Cell and Its Application in Immunomodulation and Reconstruction," Publication No. U.S. 2012/0128636 A1; Le et al. in a United States patent application publication, entitled "Pharmaceutical Compositions Comprising Gingiva Derived Mesenchymal Stem Cells and Methods of Treating Inflammation, Wound Healing and Contact Hypersensitivity," Publication No. U.S. 2013/0295058 A1; and Shi et al. in a Patent Cooperation Treaty (PCT) patent application publication, entitled "A Composition of Mesenchymal Stem Cells," Publication No. WO 2014210037 A2.

This disclosure also relates to a method of preparation of the composition ("preparation method").

One example of the preparation method may comprise obtaining a tissue, separating the tissue into cells, sorting a stem cell, and preparing a composition comprising the stem cells.

The tissue may be any tissue that contains a stem cell. For example, the tissue may comprise a mammalian tissue. In one example, the tissue may comprise a bone marrow tissue. In another example, the tissue may comprise a gingival tissue. The mammal may be a human. The mammal may be a non-human animal. For example, the mammal may be a non-human primate, a horse, a sheep, cattle, a hog, a dog, a cat, and a goat. The tissue may comprise a stem cell. The stem cells may be any stem cells. For example, the stem cells may be any stem cells disclosed above.

Another example of the preparation method may comprise obtaining a plurality of tissues, separating these tissues into cells, sorting the stem cells, and using the stem cells in the preparation of the composition. In this example, the plurality of tissues may comprise tissues obtained from different tissues of a mammal. For example, the first tissue may be a bone marrow tissue and the second tissue may be a gingival tissue.

The tissue may be obtained from a mammal that undergoes the treatment. In this method, the treatment may be an autologous stem cell treatment. The tissue may be obtained from a mammal other than the mammal that undergoes the treatment. In this method, the treatment may be an allogeneic stem cell treatment. Combination of said treatment methods may also be applied. For example, the treatment may comprise an autologous stem cell treatment and an allogeneic stem cell treatment.

The separating the tissue into cells may be done by a mechanical method, a chemical method, or a combination of a mechanical and a chemical method.

Examples of the mechanical method may be mincing, shredding, filtering, and the like. In other examples, the tissue may be separated into cells by using homogenizers, ultrasonicators, ball mills, and the like. A combination of these mechanical methods may also be used to have separated cells.

Examples of the chemical method may be digestion of the tissue by using acids, bases, and enzymes. For example, a collagenase and a dispase may be used to digest the tissue. The collagenase may be collagenase type I. The dispase may be dispase II. A combination of these chemical methods may also be used to have separated cells.

The preparation method may further comprise preparing cell suspensions from the digested tissue by using a mechanical method. An example of such method may be filtering the digested tissue to obtain cell suspensions. The cell suspensions may be single-cell suspensions. For example, single-cell suspensions may be obtained by passing the digested gingival tissue through a 70-micrometer trainer.

Culturing the separated cells may comprise providing a solid surface, seeding the cells on the solid surface, culturing the seeded cells, and thereby obtaining a culture comprising cells that may be adherent to the solid surface ("adherent cells") and cells that may not be adherent to the solid surface ("non-adherent cells").

The solid surface may be a surface of any solid article suitable for culturing cells. For example, it may be a wall of a culture vessel. The culture vessel may be any desired culture vessel. For example, the culture vessel may be a petri dish or a cell-culture dish. The solid article may also be a bead or a particle. The solid article may have any desired size. For example, it may be a nano- particle.

The cell may be seeded using a solution. The solution may comprise a medium suitable for culturing the mammalian cell. An example of such medium may be a-MEM manufactured by Invitrogen (Carlsbad, CA). The solution may further comprise fetal bovine serum (FBS), L-glutamine, 2-mercaptoethanol, penicillin, and streptomycin.

The preparation method may further comprise eliminating from the culture the cells that are not adherent to the solid surface. For example, the culture may be washed using PBS or other suitable buffer to eliminate from the culture the cells that are not adherent to the solid surface.

The adherent cells may further be cultured, for example, in the same conditions disclosed above.

The preparation method may further comprise dissociating from the solid surface the cells that may be adherent to the solid surface. The adherent cells may be dissociated from the solid surface by using an enzyme. The enzyme, for example, may be trypsin.

The preparation method may further comprise expanding the cultured cells. For example, the expanding the cultured cells may comprise doubling stem cells by repetitively re-seeding (passaging) them using the preparation methods disclosed above.

As disclosed in the above examples, the preparation method may comprise separating the tissue into cells and sorting the stem cells. Some examples of suitable methods for separating the tissue into cells and the sorting the stem cells have been previously disclosed. For example, for separating gingival tissue into cells, and sorting and isolating GMSCs, see Le et al. in a United States patent application publication, "Gingiva Derived Stem Cell and Its Application in Immunomodulation and Reconstruction" U.S. 2012/0128636 A1; and Shi et al. in a Patent Cooperation Treaty (PCT) patent application serial number PCT/US14/43918, "A Composition of Mesenchymal Stem Cells".

The preparation method may further comprise culturing the separated cells before sorting the stem cells. The sorting may comprise sorting cells that are positive for a stem cell marker, e.g., using an antibody labeled with a suitable label such as fluorescein isothiocyanate.

This disclosure also relates to a method of administration of the composition comprising a stem cell, IL-1RA, an IL-1 RA substitute, a stem cell and IL-1 RA, a stem cell and an IL-1 RA substitute, a stem cell and IL-1 RA and an IL-1 RA substitute, or a combination thereof ("administration method"). The IL-1 RA can be intracellular IL-1 RA present in a stem cell. The administration method may comprise any method suitable for administration of a cell or a tissue. The administration method may comprise any method suitable for administration of IL-1 RA. The composition comprising the stem cells and/or IL-1 RA suitable for the treatment purposes may be administered (or delivered) in various ways. For example, they may be infused, injected at various sites, or surgically implanted. The method may comprise administration of a stem cell and an IL-1 RA substitute, either as component of a single composition or as separate compositions. When administered as separate compositions, the compositions are administered to provide for overlap in the therapeutic activity of the stem cell and the IL-1 RA substitute. As described herein, the stem cell can include intracellular IL-1 RA which can be released when the stem cell encounters pro-inflammatory stimuli (e.g., pro-inflammatory cytokines).

Suitable compositions include, for example, a stem cell and/or IL-1 RA suspension in a liquid (preferably aqueous) medium, or the stem cells in aggregated form with or without solid supports or encapsulating materials. The liquid medium may be a pharmaceutically acceptable liquid medium. The liquid medium may be suitable for injection. Suitable liquid media are generally well known and include, for example, normal saline (with or without glucose and/or potassium), Ringer's solution, Lactated Ringer's solution, and Hartmann's solution.

The composition may be administered in an amount effective to treat a mammal. The amount of the stem cells, IL-1 RA, IL-1 RA substitute, or a combination thereof in the composition ("dose") may be an amount effective to treat a mammal. "A combination thereof" here means any combination of the stem cell, IL-1RA, IL-1RA substitute. Examples of such combinations are disclosed above. The amount and/or concentration of stem cells, IL-1 RA, IL-1 RA substitute, or a combination thereof in the composition can be selected to provide for convenient administration of an amount (of the composition that is acceptable for the mammal being treated). For example, a liquid composition that contain a high concentration of the stem cells, IL-1 RA, IL-1 RA substitute, or a combination thereof is suitable for injection of a relatively small volume into solid tissue, while a liquid composition that contains a lower concentration of the stem cells, IL-1 RA, IL-1 RA substitute, or a combination thereof may be advantageous for administration by intravenous infusion. Suitable liquid compositions can contain, for example, about 1×10¹ stem cells/mL to about 1×10¹⁰ stem cells /mL, or about 1×10⁵ stem cells/mL to about 1×10⁷ stem cells/mL, or about 1×10⁶ stem cells/mL to 5×10⁶ stem cells/mL. Aggregated or solid compositions can contain, for example, about 1×10¹ stem cells/mg to about 1×10¹⁰ stem cells/g.

The composition may further comprise a carrier. The carrier may be suitable to host the stem cells and/or IL-1 RA. Example of the carrier may be in the form of matrices, tissues, fibers, beads, or other materials.

The composition may comprise stem cells in an amount in the range of 1×10¹ stem cells/kg to 1×10¹⁰ stem cells/kg, or 1×10⁵ stem cells/kg to 1×10⁷ stem cells/kg, or 1×10⁶ stem cells/kg to 5×10⁶ stem cells/kg.

For therapeutic purposes, an effective amount of the composition is administered to a mammal in need thereof. An "effective amount" in an amount that produces the desired effect under the conditions of administration, for example, an amount sufficient to treat a wound, inhibit inflammation, inhibit an immune or autoimmune response, inhibit bone loss, inhibit tumor growth or metastisis, promote wound healing, or promote colony formation, osteogenesis or adipogenesis. The exact dosage of stem cells and/or IL-1 RA to be administered may dependent upon a variety of factors, including the age, weight, and sex of the mammal, the disease being treated, and the extent and severity thereof. A clinician of ordinary skill can determine the appropriate dose and method for administration based on these and other considerations.

Any combination of products such as compositions comprising stem cells and/or IL-1RA, methods of their preparation, and methods of their use that are described herein may also be made and followed.

Other examples of this disclosure are as follows.

### Example 1. IL-1RA may play a critical anti-inflammatory role in oral wound healing.

In this example, both gingiva derived mesenchymal stem cells (GMSCs) and skin derived mesenchymal stem cells (SMSCs) were cultured form the same mouse and the culture supernatant was collected for a cytokine array. The cytokines derived from these two types of cells had a significantly different profile (**FIG. 1**).

Among these differences, one of the anti-inflammatory cytokine, IL-1RA showed a significantly higher expression level in GMSCs when compared with SMSCs. Inflammation, the first stage of wound repair, occurs immediately after tissue damage. Meanwhile, components of the coagulation cascade, inflammatory pathways and immune system are required to prevent ongoing blood losses and infection (Gurtner et al., 2008). A key damaging player in the pro-inflammatory response in numerous and diverse injuries is the cytokine interleukin-1 (IL-1) (Allan et al., 2005). Within the IL-1 ligand family, IL-1RA is a natural receptor antagonist (Dinarello, 2010). IL-1 RA binds IL-1 R1 with an affinity higher than that of IL-1 but fails to recruit the IL-1RAcP (Garlanda et al., 2013), therefore, inhibit the downstream pathway being activated.

The western blotting assay was used to show that SMSCs express less IL-1RA when compared with GMSCs in wild-type control mice (**FIG. 2A**), while the enzyme-linked immunosorbent assay (ELISA) result showed that GMSCs also released higher level of IL-1RA in culture supernatant compared with SMSCs (**FIG. 2B**).

This finding was also tested on the human cells. Human GMSCs also showed an elevated expression of IL-1RA both in cells and in culture supernatant when compared with the human SMSCs (**FIG. 2C**). This finding was also confirmed on human GMSCs. When fas siRNA was used to knock down the FAS expression on human GMSCs, the IL-RA expression level in cell increased dramatically (**FIG. 2D**).

To further confirm the IL-1RA expression profile in *in vivo* scenario, the NESTIN was used as a marker to label the MSCs as previously described (Mendez-Ferrer et al., 2010) to localize the MSCs *in vivo.* The immunofluorescence staining showed NESTIN⁺ mesenchymal stem cells were positive with IL-1 RA expression in gingiva while rarely observed in skin (**FIG. 3A**). Statistical analysis showed that in gingiva/mucosa, there are significantly higher number of IL-1RA⁺ and NESTIN⁺ mesenchymal stem cells when compared with skin (**FIG. 3B**).

In order to confirm the contribution of IL-1 RA in the GMSCs to gingiva mucosa wound healing, the mucosa wound on IL-1 RA KO mouse and WT mouse were compared. An about 1.5 mm in diameter of skin was punched on the palate of the mice and the mice was fed with the soft food. The mice were sacrificed after a predetermined time and the wound area was calculated for statistical analyses. The data showed that wound healing was significantly delayed in IL-1 RA KO mice at each time point (about 5 days after the wound was formed) (**FIG. 4A**). Statistical analysis confirmed what histological observation indicated (**FIG. 4B**).

In order to exclude the effect of the IL-1 RA secreted by other types of immune cells in a wound at a later stage, the IL-1 RA neutralizing antibody was also used to block the IL-1 RA in wild-type mice before the wound formation (**FIG. 5A**). The results were consistent with those shown with the IL-1 RA KO mouse, which suggests that the blockage of the IL-1 RA in the beginning stage of the wound was enough to delay the wound healing in mucosa (**FIG. 5B**). Statistical analysis showed that during each time course, the IL-1RA neutralizing antibody significantly delayed the wound healing process in wild type mice (**FIG. 5C**). It was also confirmed that IL-1RA knockout mice showed delayed skin wound healing (**FIG. 6**).

### Example 2. FAS may serve as a key molecule in the secretion of IL-1RA

There are four isotypes of IL-1RA found up till now. One of the isotype is a secreted soluble form of IL-1 RA with a signaling peptide at the beginning of the protein, which can actively release from cells. In addition to secreted soluble IL-1RA, there are also intracellular isoforms, which are usually considered a reservoir of IL-1 RA, to be released upon cell death, limiting the proinflammatory action of tissue damage (Muzio et al., 1995).

The real-time PCR data showed that GMSCs and SMSCs had same mRNA expression level of soluble IL-1 RA (**FIG. 7A**), while GMSCs had a significantly higher mRNA expression level of intracellular IL-1 RA when compared with SMSCs (**FIG. 7B**).

As shown previously that fas KO (Ipr) mice may have deficiency in cytokine release on bone marrow mesenchymal stem cells (BMMSCs). Therefore, two cell types, wild-type GMSCs (WT) and Fas deficient GMSCs (Ipr) were tested for their cytokine release profile. It was shown that WT GMSCs secreted a higher level of IL-1 RA in culture supernatant than Ipr GMSCs by ELISA (**FIG. 8A**). Also, the western blotting assay showed that Ipr GMSCs remained more IL-1 RA in cytoplasm (**FIG. 8B**). To further verify this finding, when the WT GMSCs were treated with fas siRNA, the IL-1 RA protein was blocked in the cell with less secreted into the culture supernatant shown by ELISA (**FIG. 8C**) and with more remained in cell as was shown by western blotting assay (**FIG. 8D**). Moreover, when FAS was over expressed in Ipr GMSCs, the IL-1 RA secretion was enhanced as more IL-1 RA was detected in the culture supernatant by ELISA (**FIG. 8E**) and less IL-1 RA remained in cells as shown by western blotting assay (**FIG. 8F**).

### Example 3. FAS may work together with CAVEOLIN1 and SNARE family member in IL-1 RA releasing.

In this example, the cell membrane transportation was investigated. Since the type of intracellular IL-1 RA does not have the signaling peptide allowing it to be automatically transported outside of the cell, it may be packaged into vesicle and transported by proper machinery system on the cell membrane. Therefore, first, a main lipid raft structure named caveolae was found. It dynamically participates in a number of cellular processes including signal transduction, lipid regulation and membrane trafficking (Parton *et al.*, 2013), which has a key protein CAVEOLIN 1 (CAV1). Besides, the Soluble NSF Attachment Protein Receptor (SNARE) family proteins may also be involved. SNARE proteins are a large protein superfamily to mediate the exocytosis of cellular transport vesicles with the cell membrane in yeast and mammalian cells (Sutton *et al.*, 1998). Some key molecules in this family were SYNTAXIN, SNAP25 and Vesicle Associated Membrane Protein (VAMP).

These key molecules were tested to see any of them work together with FAS to mediate the IL-1RA release. First, the sucrose gradient centrifuge methods were used to determine whether IL-1 RA was packaged within certain size of the vesicles. As show in **FIG. 9**, IL-1RA appeared in fractions #1-#4, which were the smallest sized fractions. It was found that the Vesicle Associated Membrane Protein 5 (VAMP5) was co-localized with IL-1RA in same fractions. The immunoprecipitation experiment was carried out to confirm the molecules functioning in complex for the IL-1RA containing vesicle releasing. The western blotting assay of the protein pulled down by FAS antibody showed that in WT GMSCs, FAS were binding with CAV1, SYNTAXIN, SNAP25, VAMP5 and IL-1RA (**FIG. 10A-10C**).

The binding was also confirmed at the cell level, as the immunofluorescence staining showed co-localization of FAS, CAV-1, SYNTAXIN and SNAP25 on WT GMSCs membrane (**FIG. 11A-11D**). To further verify the complex components, an immunoprecipitation experiment was carried out to pull down every of the previously detected molecule to investigate their binding details. As shown by western blotting assay, when the protein was pulled down by SYNTAXIN antibody, FAS, CAV1, SNAP25, VAMP5 and IL-1RA were found to bind together in WT GMSCs while not in *lpr* GMSCs (**FIG. 11E, 11F**). However, when the CAV-1 antibody was used to pull down the target protein, it was found that all the other molecules remained the same trend as seen in the previous immunoprecipitation except SYNTAXIN, which showed a similar binding in both WT and *lpr* GMSCs (**FIG. 10B**). The result indicated that in WT and Ipr GMSCs, the CAV1 can bind with the SYNTAXIN indifferently.

The CAV1 protein can be phosphorylated at Tyr14 and this phosphorylation has been linked to membrane internalization, endocytosis, focal adhesion stability and dynamics, directional cell migration and extra cellular matrix remodeling (Parton et al., 2013 and del Pozo et al., 2005). Therefore, the phospho-CAV1 expression was tested in both WT and Ipr GMSCs. It was found that there is a much higher expression of the phospho-CAV1 in *lpr* GMSCs as shown by western blotting assay (**FIG. 11E**).

In previous studies, CAV1 was usually believed to be phosphorylated by Src family proteins. However, when the universal phospho-Src expression was tested in both WT and *lpr* GMSCs, there was no difference (**FIG. 11E**). Since the *lpr* GMSCs have the mutation on *fas* gene, FAS may be the key mediator in this phosphorylation. FAS related phosphatase was searched and found FAS Associated Phosphatase-1 (FAP-1) may function in CAV1 dephosphorylation. Though the expression of the FAP-1 in both WT and *lpr* GMSCs remained the same (**FIG. 11E**), the siRNA was used to knock down the *fap-1* gene expression to verify its function in cell. When the FAP-1 expression was knocked down in WT GMSCs, the phospho-CAV1 expression was increased but the phospho-Src remained unchanged. Consequently, the IL-1RA concentration in cytoplasm was increased (**FIG. 11F**). The immunocytofluorescence staining was also used to confirm the phospho-CAV1 expression on the cell level.

As shown by the immunofluorescence staining, *lpr* GMSCs expressed higher level of phospho-CAVEOLIN-1 (**FIG. 12A**). Previous studies showed that the CAV1 phosphorylation may cause the alteration of the caveolae structure in order to fulfill the physiological functions. The immunofluorescence staining also showed the altered the caveolae distribution in *lpr* GMSCs (**FIG. 12B**). In this example, the western blotting assay was used to analyze the proteins by pulling down by phospho-CAV1 immunoprecipitation. FAS, SYNTAXIN, IL-1RA and VAMP5 were rarely found bound with the phospho-CAV1 in both WT and *lpr* GMSCs. However, more SNAP25 was bound with phospho-CAV1 in *lpr* GMSCs when compared with WT GMSCs (**FIG. 10C**). Thus, these results showed that the phosphorylation of CAV1 would bind with the SNAP25, therefore, inhibiting the SNAP25 binding together with SYNTAXIN to trigger the machinery complex to release the IL-1RA containing vesicles.

To further confirm this complex on the functional level, the siRNA was used to knock down *cav1, syntaxin, snap25* and *vamp5* gene expression in WT GMSCs. The western blotting assay showed that knocked down protein expression of the CAV1, SYNTAXIN, SNAP25 and VAMP25 all leaded to the increase of the IL-1RA expression in cells (**FIG. 11B**). And these were also confirmed by ELISA assay, which showed a decrease of the IL-1RA concentration in cell culture supernatant (**FIG. 11D****).**

### Example 4. IFN-γ signaling may modulate the IL-1RA containing vesicle release.

The release mechanism of pathophysiological microenvironment was investigated in this example. First, regulation of the IL-1RA releasing through FAS signaling was tested. The WT GMSCs were tested with different concentration of the FAS activating antibody to activate the FAS signaling. There was no change of the phospho-CAV1, phospho-Src and IL-1RA expression level in cells as shown by western blotting assay (**FIG. 13A**).

Since the SNARE family members, which were involved in microvesicle release, were being activated by calcium influx in neuron system, and then, calcium influx control of the IL-1RA release was tested. Western blotting assay confirmed that two major calcium channel structure proteins functioning in neuron transmitter release - Caᵥ2.1 and Caᵥ2.2 showed very low expression in WT GMSCs (**FIG. 13B**). Then, ω-Agatoxin TK and ω-Conotoxin GVIA, the antagonist of these two proteins, were used to block their function in cells to investigate change of the IL-1RA expression levels. Western blotting data showed that the blockage of both Caᵥ2.1 and Caᵥ2.2 did not alter the IL-1 RA expression level in cells while the FAS expression level also remained same (**FIG. 13C**). The ELISA assay was used to confirm the IL-1 RA concentration in the cell culture supernatant, which showed no change in any of the antagonist (**FIG. 13D**).

The environmentally derived signaling may modulate the IL-1RA release. The cell was treated with the TGF-β, which was abundant in wound tissue. It was found that the TGF-β signaling pathway was activated in WT GMSCS as the phospho-SMAD2 expression up-regulated. However, the IL-1RA expression level did not change in cells as shown by western blotting (**FIG. 13E**). This was also confirmed by ELISA assay, which showed that the IL-1 RA concentration remained unchanged in cell culture supernatant after TGF-β treatment (**FIG. 13E**).

Another important category of signaling was that the inflammatory cytokines emerged after the wound creation. Therefore, the most common inflammatory cytokines IFN-γ and TNF-a were chosen to test their function on IL-1 RA release. It was found that when WT GMSCs were treated by IFN-γ and TNF-a, both FAS and FAP-1 expression were up-regulated. Consequently, the phospho-CAV1 level decreased (**FIG. 13F**). The ELISA assay showed that after the treatment of IFN-γ and TNF-α, the IL-1RA concentration in the cell culture supernatant increased (**FIG. 13G**). Though TNF-a can also trigger the IL-1RA release in WT GMSCs, when compared with the IFN- γ, its stimulation effect was mild.

### Example 5. IL-1RA substitute may ameliorate the skin wound healing

In this example, treatment of skin wounds by the IL-1RA substitute was investigated. An example of IL-1RA substitute may be Kineret^{®} (anakinra), which is a recombinant, nonglycosylated form of the human IL-1RA. Anakinra differs from native human in that it has the addition of a single methionine residue at its amino terminus. It may be produced by recombinant DNA technology using an *E coli* bacterial expression system. The composition may comprise IL-1 RA substitute with a concentration, for example, in the range of 1 mg to 1,000 mg per mL of the composition; or 10 mg to 200 mg per mL of the composition. The composition may comprise IL-1RA substitute with a concentration, for example, in the range of 1 mg to 1,000 mg per gram of the composition; or 10 mg to 200 mg per gram of the composition.

Wounds on dorsal skin of the mice were formed by using a 4 mm diameter punch and the IL-1RA substitute was infused around the wound site about 1 hour after the wound formation. The samples were collected and analyzed at different time points (**FIG. 14A**). As the data showed, the skin wounds healed much faster in the IL-1RA treated group when compared with the saline treated control at each time point (**FIG. 14B**).

The statistical analysis of the results indicated the IL-1RA substitute significantly improved the wound healing process in WT mice (**FIG. 14B**). The samples were further analyzed by histological sectioning using hemotoxylin and Eosin (H&E) staining. The results showed less inflammatory cells infiltration and better collagen deposition in the IL-1RA treatment group (**FIG. 14C**).

The skin fibroblast method was used to investigate contribution of IL-1 RA to the wound healing. The skin fibroblast was treated with IL-1β or IL-1β together with IL-1RA. The western blotting assay showed that the skin fibroblast, when treated with IL-1β, had an elevated NF-κB signaling pathway leading to SMAD7 production, which inhibited the TGF-β pathway, while the administration of IL-1 RA can antagonize the IL-1β function (**FIG. 15A**). Also, the TGF-β reaction reporter plasmid was transfected into the skin fibroblast to confirm this finding. The luciferase assay of TGF-β₁ signaling activity showed that the IL-1β largely inhibited the TGF-β₁ effect on the skin fibroblast. However, IL-1RA significantly rescued the inhibition (**FIG. 15B**).

The histological section of the wound sites on both WT and IL-1 RA KO mice were also compared. The IL-1RA function on the rabbit ear scar model was also tested. About 6 mm wound was punched down to the cartilage on ventral side of the rabbit ear. A predetermined concentration of the Kineret^{®} (anakinra) was administrated around the wound site about 1 hour after wound creation as was done in dorsal skin. Intriguingly, less scar area and even better aligned collagen deposition were observed in the group of rabbit treated with low dosage of IL-1RA. Although high dosage of IL-1RA treatment slightly compromised this effect, it still showed significantly better treatment effect when compared with the saline treated control group (**FIG. 16A**). The statistical analysis confirmed that the IL-1 RA substitute significantly reduced the scar formation in rabbit ear model (**FIG. 16B**).

### Example 6.

In this disclosure, role of gingiva mesenchymal stem cells (GMSCs) in tissue wound repair was investigated. GMSCs may be stimulated by the adjacent inflammatory environment to release increasing amount of IL-1RA to counterbalance the exaggerated pro-inflammatory response and minimize the side effects of inflammation during the wound healing.

This function was mediated by FAS receptor, which was known for long time as a death-signaling molecule. By association of FAP-1, FAS maintained the key cell membrane trafficking structure caveolae in an un-phosphorylated status to function properly with SNARE family in controlling the IL-1 RA containing vesicle release. Notably, this mechanism may not only affect the IL-1RA but also various other cytokines as the cytokine array data showed a complete different profile for the FAS knockout cells. These results suggested that the IL-1RA drug may be used in improving cutaneous wound healing and reducing the scar formation.

MSCs are multipotent cells, which can differentiate into different cell types, such as chondrocytes, osteoblasts, and adipocytes (Pittenger *et al.*, 1999). Recent studies have also suggested that MSCs may influence immune and inflammatory responses (Uccelli *et al.*, 2008). There may be a small number of dormant stem cells residing in almost all tissues that can be activated or migrate to sites of damaged tissue contributing to its repair (Phinney *et al.*, 2007).

Since MSCs interact closely with various stromal cells and inflammatory cells on the site of damage by their immunosuppressive character (Sasaki *et al.*, 2008 and Wu *et al.*, 2007), MSC-derived trophic factors play an important role in these cell-to-cell interplays. In animal models, MSC-conditioned medium could, to some extent, mimic the therapeutic effects of MSCs in enhancing wound healing, and improving cardiac function following myocardial infarction (Chen *et al.*, 2008; Gnecchi *et al.*, 2005 and Timmers *et al.*, 2011).

In this disclosure, GMSCs had strong icIL-1RA expression in ground status. These natural antagonists can be released and contribute to the mild Interleukin-1 (IL-1) based inflammatory response in the gingiva/mucosa tissue, resulting in healing of their wounds. The IL-1 RA knockout mice showed a significantly delayed wound healing. When IL-1 RA substitutes were applied to the cutaneous wound, they also improved the wound healing process. On a rabbit ear scar model, the scar formation was reduced when the IL-1 RA applied shortly after the wound creation. These findings showed that gingiva/mucosa had a much-regulated inflammatory response during injury, and the regulatory effect largely depends on the GMSCs' innate function of releasing the anti-inflammatory cytokines.

Tissue wound usually initiates the activation of immune and inflammatory cascade. Acute tissue damage is usually followed by immediate inflammation (Medzhitov *et al.*, 2008). Cellular components released from necrotic cells and microvasculature damage lead to enhanced vasopermeability and infiltration of immune cells, which are the main source of inflammatory cytokines (Luster *et al.*, 2005 and Eming *et al.*, 2007). The initial studies on the immunomodulatory effects of MSCs produced conflicting data (Uccelli *et al.*, 2008), suggesting that the immunosuppressive property of MSCs could be affected by specific damage/disease-related tissue microenvironments.

Once MSCs have encountered the microenvironment of injured tissues, many factors, including inflammatory cytokines such as IFN-γ, TNF-α, IL-1, toxins of infectious agents and hypoxia can stimulate them to release various growth factors, including transforming growth factor-b (TGF-β), epidermal growth factor (EGF), fibroblast growth factor (FGF), platelet-derived growth factor (PDGF), vascular endothelial growth factor (VEGF), keratinocyte growth factor (KGF), hepatocyte growth factor (HGF) and insulin growth factor-1 (IGF-1) (Shi *et al.*, 2012; Aguilar *et al.*, 2009 and Hung *et al.*, 2012).

The multitude of paracrine factors may provoke tissue-resident cells such as fibroblasts to promote wound healing through their secretion of extracellular matrix and matrix metalloproteinase. Several studies indicated that the priming of the MSCs by inflammatory cytokines is essential for MSC-mediated immunosuppression. Other studies also showed that pretreatment with inflammatory cytokines can enhance the therapeutic effect of MSCs (Krampera *et al.*, 2006; Sheng *et al.*, 2008 and Mougiakakos *et al.*, 2011).

These data indicated the importance of the local inflammatory condition in regulating the plasticity of MSCs. Up to date, little was known about how these inflammatory stimulus function on MSCs. The limited reports on the mechanism of this type of MSCs "licensing" showing it was in a nuclear factor (NF)-κB-dependent manner.

In this disclosure, it was shown that upon the challenge by the IFN-γ, both FAS and its associate phosphatase (FAP-1) were upregulated through JAK/STAT 1 pathway to maintain the important membrane structure caveolae in an unphosphorylated form. This unphosphorylated status helped the SNARE machinery system within the caveolae structure to function properly on the secretion of IL-1RA containing vesicle.

Tissue injury initiates a complex set of events, which finally lead to wound repair. The wound healing process begins with inflammation. Studies using various models have shown that when compared to similar wounds in skin, wound healing in oral mucosa and gingiva is markedly faster, resulting to significantly reduced scar formation.

The reason for the preferential oral mucosal wound healing may in part depend on the milder and shorter inflammatory response in oral mucosa (Glim *et al.*, 2013; Szpaderska *et al.*, 2003 and Mak *et al.*, 2009). Among all the pro-inflammatory factors immediately emerging around the lesion site, interleukin-1 (IL-1) family members are the first of which to be induced and detected (Hübner *et al.*, 1996). IL-1 is a prototypical proinflammatory cytokine that stimulates both local and systemic responses with detrimental effects in skin lesions, inflammatory arthritis, fever and leukocytosis. However, these responses are also essential in the control and resolution of infections (Miller *et al.*, 2006). Some of these effects are indirect, mediated through the induction of downstream cytokines and other inflammatory mediators (Dinarello *et al.*, 1996).

In addition, IL-1 is also involved in the regulation of adaptive immune responses by inducing the differentiation of type 17 T-helper cells and the production of IL-17 in mice and humans (Chung *et al.*, 2009 and Acosta-Rodriguez *et al.*, 2007). The proinflammatory activities of IL-1 are strictly controlled by several endogenous inhibitors, such as the first described naturally occurring antagonist IL-1RA.

IL-1RA binds tightly to IL-1R1, thereby blocking binding of IL-1α and IL-1β. In the presence of IL-1RA, the reduction of IL-17A and IL-22 is 82% and 71%, respectively (van de Veerdonk *et al.*, 2012). Moreover, unlike IL-1α and IL-1β, receptor-bound IL-1RA does not allow recruitment of IL-1RAP (Greenfeder *et al.*, 1995), thus protect the downstream signaling being activated.

It was shown that the balance between IL-1 and IL-1 RA determines the overall inflammatory response (Arend, 2002). Deficiency of IL-1RA in mice results in spontaneous and lethal arteritis, destructive arthritis, and psoriatic-like skin lesions, as well as increased susceptibility to carcinogenesis (Horai *et al.*, 2000 and Nicklin *et al.*, 2000).

Children born with a genetic deficiency of IL-1 RA or functional inactive IL-1RA suffer from severe systemic and local inflammation, including pustular skin eruptions, vasculitis, osteolytic lesions, and sterile osteomyelitis (Aksentijevich *et al.*, 2009 and Reddy *et al.*, 2009). IL-1RA is produced as four different isoforms by using different first exons and alternative mRNA splicing and translation initiation codons. One isoform is secreted (sIL-1RA), whereas the three others lack a consensus leader peptide and remain intracellular (icIL-1RA1, icIL-1RA2 and iclL-1 RA3). Intracellular forms of IL-1RA are believed to release following cell death and act extracellularly on IL-1R1, similarly to the secreted form (Palmer *et al.*, 2007).

In this disclosure, it was found that besides the cell death, proinflammatory cytokines such as IFN-γ can stimulate the GMSCs to release high level of icIL-1RA into the wound area, counterbalance the IL-1 signaling. Therefore, this may partially explain the reason of the mild inflammation observed after gingiva wound injury.

It was also shown that if the GMSCs are blocked on this response, either through the inhibition of the IFN-γ or IL-1 RA, gingiva wound healing may be significantly delayed when compared with the WT control. These results comply with the study of skin wound on IL-1RA knockout mice (Ishida *et al.*, 2006), proving that the newly found IL-1 RA releasing character on GMSCs is decisive in gingiva wound healing.

FAS (CD95/APO-1) is a member of the tumor necrosis factor (TNF) superfamily (Trauth *et al.*, 1989 and Yonehara *et al.*, 1989). Studies on the FAS system focused primarily on its role in apoptosis. FAS is a prototypical death receptor with an intracellular "death domain" (DD), which is essential for transduction of the apoptotic signal (Locksley *et al.*, 2001). Upon ligation by FAS-Ligand, FAS receptors multimerize and recruit FAS-associated death domain (FADD) through DD-DD interactions (Scott *et al.*, 2009). FADD in turn recruits pro-caspase-8 through death effector domain (DED) forming death inducing signaling complex (DISC) (Tibbetts *et al.*, 2003).

Depending on different signaling activation, apoptosis proceeds through either extrinsic or intrinsic apoptosis pathway (Scaffidi *et al.*, 1998). Recent studies suggest, however, FAS may mediate other cellular non-apoptotic responses such as suppression of T-cell activation (Kovacs *et al.*, 1995), promoting the proliferation of CD3-activated primary T cells (Alderson *et al.*, 1993) and a variety of non-immunological cells including, fibroblasts, tumor cells, hepatic stellate cells and neural cells (Freiberg *et al.*, 1997; Chen *et al.*, 2010; Desbarats *et al.*, 2000; Reinehr *et al.*, 2008 and Lambert *et al.*, 2003).

In addition, FAS system can promote cell migration and invasion, in particular in apoptosis-resistant malignant cells (Barnhart *et al.*, 2004; Li *et al.*, 2009; Trauzold *et al.*, 2005 and Letellier *et al.*, 2010). It was also shown that FAS signaling could trigger inflammation by promoting the inflammatory factors expression in different types of cells (Brint *et al.*, 2013). Such factors may in turn recruit inflammatory cells, exacerbating the inflammatory process. FAS-induced inflammatory factor production predominantly involves the activation of transcription factor NF-κB, mitogen-activated protein kinase (MAPK) pathways (Zhang *et al.*, 2009 and Palao *et al.*, 2006) and the cross talk between toll-like receptor (TLR) family (Ma *et al.*, 2004).

Some studies suggested that one of the key mechanisms of regulating the apoptotic or non-apoptotic pathway was the FAS receptor internalization. During the apoptotic process, DISC has been shown to continue binding with the internalized FAS receptor, while inhibition of FAS receptor internalization engages it to induce pro-survival pathways (Eramo *et al.*, 2004). Caveolae and CAVEOLIN-1 (CAV1) have been known to involve in the internalization of FAS receptor complex, and blockade of CAV1-mediated endocytosis diminishes cell apoptosis (Gajate *et al.,* 2001; Zhu *et al.*, 2009 and Guo *et al.*, 2009). Caveolae, with CAV1 as its principle component, are the main structure of lipid rafts that are distinct cholesterol- and sphingomyelin-rich, flask-shaped invaginations (60-80 nm) of the plasma membrane. It is dynamically participate in a number of cellular processes including signal transduction, lipid regulation and membrane trafficking (Parton *et al.*, 2013).

Phosphorylation of CAV1 at Tyr14 has also been linked to membrane internalization, endocytosis, focal adhesion stability and dynamics, directional cell migration and extra cellular matrix remodeling (Parton *et al.*, 2013 and del Pozo *et al.*, 2005). However, the functional switch of CAV1 between its phosphorylated and un-phosphorylated form is not yet clear.

It was previously found that FAS receptor contributed to cytokine secretion as the FAS mutant *lpr* bone marrows mesenchymal stem cells (BMMSCs) showed a significantly altered cytokine releasing profile when compared with the WT control (Akiyama *et al.*, 2012).

In this disclosure, it was shown that adding FAS activating antibody did not influence the IL-1RA releasing of GMSCs, which suggested that non-apoptotic FAS pathway played an anchoring role in cytokine releasing. When under the inflammatory environment, FAP-1 could be induced and function together with FAS receptor to maintain the CAV-1 in un-phosphorylated form,, which thereby inhibit the FAS mediated death pathway being activated through internalization, meanwhile keeping the SNARE complex to be properly functioning in transporting the IL-1 RA containing vesicle. These findings to some extent explained the mechanism of how MSCs can escape from the immediate elimination by the innate immune system when infused into circulation for cell therapy.

The discovery that MSCs can modulate the inflammation as well as the host immune system has revolutionized the stem cell therapy. Although most of current investigations focus on the immunomodulatory capability of MSC populations expanded *in vitro*, the physiological roles of tissue-resident MSCs in immunomodulation have yet be elucidated (Wang *et al.*, 2014). Understanding the function of the tissue-resident MSCs will undoubtedly bring new insight into these unique cells in various pathophysiological conditions. The intercellular communication is generally achieved by direct cell-to-cell contact, secreted paracrine molecules or by the exchange of the extracellular vesicles.

In this disclosure, it was shown that the *in situ* GMSCs were stimulated by the wound inflammation to secrete more IL-1 RA containing vesicles to balance the inflammatory environment. IL-1 RA may not be the only peptide, which is involved in the complicated wound healing process.

A wealth of studies showed that vesicles may also contain genetic information such as DNA, mRNA and miRNAs (Valadi *et al.*, 2007). Therefore the microvesicle release system on GMSCs, disclosed here, may also apply to various other containing contents.

Investigations in the past few years have provided solid evidence for the function of MSCs in the treatment of immunological disorders. However, there are still important concerns about the therapeutic use of these unique cells such as the origins of the tissues, the use of autologous or allogeneic MSCs, etc. need to be clarified. The understanding of the detailed physiological role of MSCs in the *in vivo* scenario will definitely do help to guide the appropriate application of MSCs and optimize to reach better modulation of inflammatory responses at different stages of disease progression.

### Example 7. Methods

The methods disclosed below were used in above examples.

**Animals.** Female C57BL/6J (stock 000664), C3H/HeJ (stock 000659), C3.MRL-*Fas^{lpr}*/J (stock 000480) and B6.129S7*-Ifng^{tm1Ts}*/*J* (stock 002287) mice were purchased from Jackson Lab. All animal experiments were performed under the institutionally approved protocols for the use of animal research (University of Southern California protocol number 11141, 11953 and 11327). Mice 2-3 months of age were used in the experiments.

**Antibodies and Reagents** Anti-runt-related transcription factor 2 (RUNX2) and -Osteocalcin (OCN) antibodies were purchased from Millipore (Billerica, MA, USA). Anti-alkaline phosphatase (ALP), -Nestin, -β-TUBUHN III, - Collagen II, -Sox9 and -Neurofilament Medium (NF-09) antibodies were purchased from Abcam (Cambridge, MA, USA). Anti-Sca-1-PE, -CD34-PE, - CD44-PE, -CD45-PE, -CD11b-PE, -CD73-PE, -CD117-PE, -CD4-PerCP, -CD25-APC, -IgG1-PE, -IgG₂ₐ-PE, -IgG_{2b}-PE, -CD3ε and -CD28 antibodies were purchased from BD Bioscience (San Jose, CA, USA). Anti-Foxp3-PE, -IL17-PE, - CD105-PE and -CD90-PE antibodies were purchased from eBioscience (San Diego, CA, USA). Purified anti-peroxisome proliferator-activated receptor γ (PPARγ), -lipoprotein lipase (LPL) and -FAS Ligand (FASL) antibodies, as well as secondary antibodies, and, were purchased from Santa Cruz Biosciences (Santa Cruz, CA, USA). Anti-β actin antibody was purchased from Sigma (St. Louis, MO, USA). EdU imaging kit was purchased from Invitrogen (Invitrogen, Carlsbad, CA, USA).

**Cell Culture.** Gingiva tissues from mouse mandibular molar region were gently separated, minced, and digested with solution containing about 2mg/mL collagenase type I (Worthington Biochemical, Freehold, NJ, USA) and about 4mg/mL dispase II (Roche Diagnostics, Indianapolis, IN, USA) in phosphate buffered saline (PBS) for about 1 hour at about 37°C. Single-cell suspensions were obtained by passing the cells through a 70-µm strainer (BD Biosciences, Franklin Lakes, NJ, USA). All nucleated cells (ANC) were seeded at 1 × 10⁶ into 100-mm culture dishes (Corning, NY, USA) with α-MEM (Invitrogen, Carlsbad, CA, USA) supplemented with about 20% FBS, about 2mM L-glutamine (Invitrogen), 55µM 2-mercaptoethanol (Invitrogen), about 100 U mL⁻¹ penicillin, and about 100µg mL⁻¹ streptomycin (Invitrogen), followed by an initial incubation for about 48 hours under about 37°C at about 5% CO₂. The cultures were washed with PBS twice to eliminate the non-adherent cells. Attached cells were cultured for another 12 days under same condition in the complete medium mentioned above.

**Histology.** To assess wound healing progress, skin and maxilla wound samples were fixed in about 4% paraformaldehyde (Sigma-Aldrich) and then decalcified (maxilla samples) with about 5 % EDTA (pH 7.4), followed by paraffin embedding. Paraffin sections (about 6 µm) were stained with hematoxylin and eosin (H&E) and analyzed by NIH ImageJ software. To perform Immunohistochemistry staining, the paraffin embedded sections were blocked with serum matched to secondary antibodies, incubated with the IL-1RA or IL4 specific antibodies (Santa Cruz Biotechnology, Inc, 1:400) at about 4°C for overnight, and then stained using VECTASTAIN UNIVERSAL elite ABC kit and ImmPACT VIP Peroxidase Substrate kit (VECTOR) according to the manufacturer's instruction.

**ELISA.** Cell culture medium was collected, and IL-1RA protein levels were analyzed by using mouse ELISA Quantikine^{®} Immunoassay kit (R&D Systems MRA00), according to the manufacturer's instructions.

**Real-Time PCR.** Total RNAs were isolated from different GMSCs by means of the TRIzol^{®} Reagent (Life Technologies, Invitrogen). RNA samples (about 1 µg) were reverse-transcribed in a Reverse Transcription system (QIAGEN). Primers used were: (*sIl-1ra*) forward, 5'-AAATCTGCTGGGGACCCTAC-3' (SEQ ID NO:1), and reverse, 5'-TCCCAGATTCTGAAGGCTTG-3' (SEQ ID NO:2); (*icIl-1ra*) forward, 5'-AGACACTGCCTGGGTGCTCCT-3' (SEQ ID NO:3) and reverse, 5'-GTTTGATATTTGGTCCTTGTAAG-3' (SEQ ID NO:4) and (*GAPDH* as an internal control) forward, 5'-GAAGGTGAAGTTCGGAGTC-3' (SEQ ID NO:5), and reverse, 5'- GAAGATGGTGATGGGATTTC-3' (SEQ ID NO:6). PCR conditions were: about 95°C for about 5 min, (about 95°C for about 10 sec, about 50°C for about 45 sec) × 40 times, and about 90°C for about 10 sec.

**Western Immunoblotting.** Cells were lysed in M-PER mammalian protein extraction reagent (Thermo) with protease and phosphatase inhibitors (Roche), and proteins were quantified using protein concentration assay (Bio-Rad Laboratories). About 20 µg of proteins were separated by SDS-PAGE and transferred to about 0.2 µm nitrocellulose membranes (Millipore). The membranes were blocked with about 5% non-fat dry milk and about 0.1% Tween-20 for about 1 hour, followed by incubation overnight with the primary antibodies diluted in blocking solution according to manufacturer's instructions. Antibodies to mouse FAS were purchased from Abcam, Cell Signaling, Millipore, and Sigma-Aldrich. The membranes were then incubated under room temperature for about 1 h in species related HRP-conjugated secondary antibody (Santa Cruz) diluted at about 1:10,000 in blocking solution. Immunoreactive proteins were detected using SuperSignal^{®} West Pico Chemiluminescent Substrate (Thermo) and BioMax film (Kodak).

**RNAi and Chemical Reagent Treatments.** GMSCs (about 0.3×10⁶) were seeded to a 60mm culture plate and treated with *Cav1* siRNA (Santa Cruz), *Fas* siRNA (Santa Cruz), *Snap25*□siRNA (Santa Cruz) according to the manufacturers' instructions. After transfection, cells were either used for protein extraction for Western immunoblotting or for live imaging. For chemical reagent treatments, serum-starved GMSCs were treated with about 200 ng/mL IFN-γ (R&D Systems), about 50 ng/mL TNF-a (Peprotech), or about 1 µg/mL TGFβ neutralized antibody (R&D systems). For Western immunoblotting, MSCs were cultured under growth medium with treatments for about 24 hours, and protein was extracted by using M-PER mammalian protein extraction reagent.

**Immunofluorescent Microscopy.** GMSCs were cultured on 2-well chamber slides (Nunc) (about 1×10³/well) and then fixed with 4% paraformaldehyde. The Chamber slides or the previous prepared histological sections were incubated with primary anti CD73 antibody (about 1:400, BD) and anti IL4Ra (about 1:400, BD) at about 4°C for overnight. And then treated with Alexafluoro 568 or Alexafluoro 488 conjugated secondary antibody (about 1:200, Invitrogen) for about 1 hour at room temperature. Finally, slides were mounted with Vectashield mounting medium (Vector Laboratories).

**Luciferase Reporter Assay.** *Tgfβ* reaction luciferase promoter reporter constructs were purchased from Addgene (plasmid 11767, Wrana *et al.*, 1992). GMSCs cultured in 6-well plates were co-transfected with 2 µg of luciferase reporter and 100 ng of Renilla luciferase expression vector to control for transfection efficiency. Forty-eight hours after transfection, cells were lysed in 1× passive lysis buffer, and luciferase activity was measured using the Dual-Glo Luciferase System (Promega) and a luminometer (Turner Biosystems).

***Il-1ra-GFP* Plasmid Construction.** *Il-1ra-GFP* expression plasmid constructs were generated by PCR using Pfu polymerase and mouse *Il-1ra* cDNA vector (GE LifeScience MMM1013-202766323) as a template. Primers containing upstream Mlul and downstream Nhel restriction sites were used to generate *Il-1ra* cDNA fragment (forward, 5'-ACGCGTATGGCTTCAGAGGCAGCCTG-3' (SEQ ID NO:7) and reverse, 5'- GCTAGCTTGGTCTTCTGGAAGTAGA-3'(SEQ ID NO:8)). Restriction digested PCR products were subcloned into pBI-GFP vector (Addgene plasmid 16623, Cahill *et al.,* 1998). All clones were confirmed by sequencing on both strands. GMSCs cultured on 2-well chamber slides (Nunc) (about 1×10³/well) were transfected with about 2 µg of *Il-1ra-GFP* expression plasmid by Lipofectamine^{®} 3000 (Invitrogen). About 48 hours after transfection, cells were kept in UNO CO₂ Microscope Stage Incubator (okolab) for confocal microscope live imaging (Leica).

**Statistics.** All experimental group sizes were chosen to ensure adequate statistical power despite the highly variable nature of the studies performed. No animal excluded, and animals were randomly assigned groups for the studies. All experiments were not performed in a blinded fashion. Data were assessed for normal distribution and similar variance between groups. Comparisons between two groups were analyzed by independent unpaired two-tailed Student's *t*-test, and the comparisons between more than two groups were analyzed by one-way ANOVA with the Bonferroni adjustment. The *p* values less than 0.05 were considered statistically significant.

Any combination of products such as compositions comprising stem cells and/or IL-1RA, methods of their preparation, and methods of their use that are described herein may also be made and followed.

In this disclosure, the indefinite article "a" and phrases "one or more" and "at least one" are synonymous and mean "at least one".

Relational terms such as "first" and "second" and the like may be used solely to distinguish one entity or action from another, without necessarily requiring or implying any actual relationship or order between them. The terms "comprises," "comprising," and any other variation thereof when used in connection with a list of elements in the specification or claims are intended to indicate that the list is not exclusive and that other elements may be included. Similarly, an element preceded by an "a" or an "an" does not, without further constraints, preclude the existence of additional elements of the identical type.

## Claims

1. A composition comprising an IL-1RA substitute and at least one human stem cell that expresses IL-1RA, wherein the human stem cell is a human gingiva-derived mesenchymal stem cell, and wherein the human stem cell further comprises a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human periosteum-derived stem cell, a human salivary gland-derived stem cell, or a combination thereof.

2. The composition of claim 1, wherein the composition comprises an effective amount of the IL-1 RA substitute and an effective amount of human stem cells such that the composition is effective in a treatment of a wound or in reducing scar formation when the composition is applied to a wound.

3. The composition of claim 1, wherein the IL-1RA substitute is anakinra.

4. A composition comprising human IL-1RA, wherein the human IL-1RA is derived from at least one human stem cell, and a human gingiva-derived mesenchymal stem cell, and wherein the human stem cell further comprises a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human periosteum-derived stem cell, a human salivary gland-derived stem cell, or a combination thereof.

5. The composition of claim 4, wherein the composition comprises an effective amount of the human IL-1RA such that the composition is effective in a treatment of a wound or in reducing scar formation when the composition is applied to a wound.

6. The composition of claim 4, wherein the composition further comprises a stem cell culture, wherein the stem cell culture further comprises a human dental pulp stem cell, a human stem cell from human exfoliated deciduous teeth, a human periodontal ligament stem cell, a human dental follicle stem cell, a human tooth germ progenitor cell, a human stem cell from an apical papilla, a human epithelial progenitor/stem cell, a human periosteum-derived stem cell, a human salivary gland-derived stem cell, or a combination thereof.

7. The composition of any one of claims 1 to 6 for use in a method of treating a wound of a human subject.

8. The composition of claim 2 or claim 5, or the composition for use of claim 7, wherein the wound is any injury to any living tissue of a mammal.

9. The composition or composition for use of claim 8, wherein the tissue is skin.

10. The composition or composition for use of claim 8, wherein the mammal is a human or a non-human animal.

## Patentansprüche

1. Zusammensetzung, umfassend einen IL-1RA-Ersatzstoff und mindestens eine humane Stammzelle, die IL-1RA exprimiert, wobei die humane Stammzelle eine humane mesenchymale Stammzelle aus dem Zahnfleisch ist, und wobei die humane Stammzelle weiter eine humane Zahnmarkstammzelle, eine humane Stammzelle aus menschlichen exfolierten Milchzähnen, eine humane Parodontalligamentstammzelle, eine humane Zahnfollikelstammzelle, eine humane Zahnkeimvorläuferzelle, eine humane Stammzelle aus einer apikalen Papille, eine humane epitheliale Vorläufer-/Stammzelle, eine humane Stammzelle aus dem Periost, eine humane Stammzelle aus der Speicheldrüse oder eine Kombination davon umfasst.

2. Zusammensetzung nach Anspruch 1, wobei die Zusammensetzung eine wirksame Menge des IL-1RA-Ersatzstoffs und eine wirksame Menge humaner Stammzellen umfasst, so dass die Zusammensetzung wirksam in der Behandlung einer Wunde oder bei der Verringerung der Narbenbildung ist, wenn die Zusammensetzung auf eine Wunde aufgetragen wird.

3. Zusammensetzung nach Anspruch 1, wobei der der IL-1RA-Ersatzstoff Anakinra ist.

4. Zusammensetzung, umfassend humanes IL-1RA, wobei das humane IL-1RA von mindestens einer humanen Stammzelle und einer humanen mesenchymalen Stammzelle aus dem Zahnfleisch stammt, und wobei die humane Stammzelle weiter eine humane Zahnmarkstammzelle, eine humane Stammzelle aus menschlichen exfolierten Milchzähnen, eine humane Parodontalligamentstammzelle, eine humane Zahnfollikelstammzelle, eine humane Zahnkeimvorläuferzelle, eine humane Stammzelle aus einer apikalen Papille, eine humane epitheliale Vorläufer-/Stammzelle, eine humane Stammzelle aus dem Periost, eine humane Stammzelle aus der Speicheldrüse oder eine Kombination davon umfasst.

5. Zusammensetzung nach Anspruch 4, wobei die Zusammensetzung eine wirksame Menge des menschlichen IL-1RA umfasst, so dass die Zusammensetzung wirksam bei der Behandlung einer Wunde oder bei der Verringerung der Narbenbildung ist, wenn die Zusammensetzung auf eine Wunde aufgetragen wird.

6. Zusammensetzung nach Anspruch 4, wobei die Zusammensetzung weiter eine Stammzellkultur umfasst, wobei die Stammzellkultur weiter eine humane Zahnmarkstammzelle, eine humane Stammzelle aus menschlichen exfolierten Milchzähnen, eine humane Parodontalligamentstammzelle, eine humane Zahnfollikelstammzelle, eine humane Zahnkeimvorläuferzelle, eine humane Stammzelle aus einer apikalen Papille, eine humane epitheliale Vorläufer-/Stammzelle, eine humane Stammzelle aus dem Periost, eine humane Stammzelle aus der Speicheldrüse oder eine Kombination davon umfasst.

7. Zusammensetzung nach einem der Ansprüche 1 bis 6 zur Verwendung in einem Verfahren zur Behandlung einer Wunde eines menschlichen Subjekts.

8. Zusammensetzung nach Anspruch 2 oder Anspruch 5 oder die Zusammensetzung zur Verwendung nach Anspruch 7, wobei die Wunde eine jegliche Verletzung eines jeglichen lebenden Gewebes eines Säugetiers ist.

9. Zusammensetzung oder Zusammensetzung zur Verwendung nach Anspruch 8, wobei das Gewebe die Haut ist.

10. Zusammensetzung oder Zusammensetzung zur Verwendung nach Anspruch 8, wobei das Säugetier ein Mensch oder ein nicht-menschliches Tier ist.

## Revendications

1. Composition comprenant un substitut d'IL-1RA et au moins une cellule souche humaine qui exprime IL-1RA, dans laquelle la cellule souche humaine est une cellule souche mésenchymateuse humaine dérivée de gencive, et dans laquelle la cellule souche humaine comprend en outre, une cellule souche de pulpe dentaire humaine, une cellule souche humaine issue de dents de lait exfoliées humaines, une cellule souche de ligament périodontique humain, une cellule souche de follicule dentaire humain, une cellule progénitrice de germe dentaire humain, une cellule souche humaine issue d'une papille apicale, une cellule progénitrice/souche épithéliale humaine, une cellule souche humaine dérivée du périoste, une cellule souche humaine dérivée de glande salivaire, ou une combinaison de celles-ci.

2. Composition selon la revendication 1, dans laquelle la composition comprend une quantité efficace du substitut d'IL-1RA et une quantité efficace de cellules souches humaines, de telle sorte que la composition soit efficace dans le traitement d'une lésion ou dans la réduction de la formation de cicatrice lorsque la composition est appliquée sur une lésion.

3. Composition selon la revendication 1, dans laquelle le substitut d'IL-1RA est l'anakinra.

4. Composition comprenant de l'IL-1RA humain, dans laquelle l'IL-1RA humain est dérivé d'au moins une cellule souche humaine, et une cellule souche mésenchymateuse humaine dérivé de gencive, et dans laquelle la cellule souche humaine comprend en outre, une cellule souche de pulpe dentaire humaine, une cellule souche humaine issue de dents de lait exfoliées humaines, une cellule souche de ligament périodontique humain, une cellule souche de follicule dentaire humain, une cellule progénitrice de germe dentaire humain, une cellule souche humaine issue d'une papille apicale, une cellule progénitrice/souche épithéliale humaine, une cellule souche humaine dérivée du périoste, une cellule souche humaine dérivée de glande salivaire, ou une combinaison de celles-ci.

5. Composition selon la revendication 4, dans laquelle la composition comprend une quantité efficace d'IL-1RA humain, de telle sorte que la composition soit efficace dans le traitement d'une lésion ou dans la réduction de la formation de cicatrice lorsque la composition est appliquée sur une lésion.

6. Composition selon la revendication 4, dans laquelle la composition comprend en outre, une culture de cellules souches, dans laquelle la culture de cellules souches comprend en outre, une cellule souche de pulpe dentaire humaine, une cellule souche humaine issue de dents de lait exfoliées humaines, une cellule souche de ligament périodontique humain, une cellule souche de follicule dentaire humain, une cellule progénitrice de germe dentaire humain, une cellule souche humaine issue d'une papille apicale, une cellule progénitrice/souche épithéliale humaine, une cellule souche humaine dérivée du périoste, une cellule souche humaine dérivée de glande salivaire, ou une combinaison de celles-ci.

7. Composition selon l'une quelconque des revendications 1 à 6, à utiliser dans un procédé de traitement d'une lésion chez un sujet humain.

8. Composition selon la revendication 2 ou la revendication 5, ou composition à utiliser selon la revendication 7, dans laquelle la lésion est tout dommage à un tissu vivant de mammifère.

9. Composition ou composition à utiliser selon la revendication 8, dans laquelle le tissu est de la peau.

10. Composition ou composition à utiliser selon la revendication 8, dans laquelle le mammifère est un humain ou un animal non humain.
